(19) Europäisches Patentamt
European Patent Office
Office européen des brevets

(11) Veröffentlichungsnummer: **0 241 003 B1**

(12) **EUROPÄISCHE PATENTSCHRIFT**

(45) Veröffentlichungstag der Patentschrift: **06.10.93**

(21) Anmeldenummer: **87105180.1**

(22) Anmeldetag: **08.04.87**

(51) Int. Cl.⁵: **C07D 405/04**, A61K 31/445, C07D 405/12, A61K 31/44, //C07F9/553,C07D211/70, C07D211/74

# (54) 4H-1-Benzopyran-4-on-Derivate, ein Verfahren zu ihrer Herstellung und ihre Verwendung als Arzneimittel.

(30) Priorität: **11.04.86 DE 3612337**

(43) Veröffentlichungstag der Anmeldung:
**14.10.87 Patentblatt 87/42**

(45) Bekanntmachung des Hinweises auf die Patenterteilung:
**06.10.93 Patentblatt 93/40**

(84) Benannte Vertragsstaaten:
**AT BE CH DE ES FR GB GR IT LI LU NL SE**

(56) Entgegenhaltungen:
**EP-A- 0 000 377**
**EP-A- 0 137 193**
**DE-B- 1 802 961**
**GB-A- 2 101 115**

(73) Patentinhaber: **HOECHST AKTIENGESELLSCHAFT**

**D-65926 Frankfurt(DE)**

(72) Erfinder: **Kattige, Samba Laxminarayan**
**17, Sai Prasad Mithagar Road**
**Mulund (East) Bombay 400 081(IN)**
Erfinder: **Naik, Ramchandra Ganapati, Dr.**
**M-4, Hoechst Executive Ouarters**
**darga Road**
**Mulund (West) Bombay 400 082(IN)**
Erfinder: **Lakdawalla, Aftab Dawoodbhai**
**11, Kapadia Mansion**
**Ground fl.**
**Dr. A. Nair Road**
**Bombay 400 011(IN)**
Erfinder: **Dohadwalla, Alihussein Nomanbhai, Dr.**
**Noman Mansion**
**139 C**
**Cumballa Hill Bombay 400 036(IN)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.6/3.3.1)

**TETRAHEDRON LETTERS, Band 79, Nr. 8, 1979, Seiten 721-724, Pergamon Press Ltd., GB; A.D. HARMON et al.: "The structure of rohitukine, the main alkoloid of amoora rohituka (Syn. Aphanamixis polystachya) (Meliaceae)"**

Erfinder: **Rupp, Richard Helmut, Dr.**
**Niladri**
**66 Nepean Sea Road**
**Bombay 400 006(IN)**
Erfinder: **de Souza, Noel John, Dr.**
**Melrose**
**62, Pali Hill**
**Bandra Bombay 400 050(IN)**

## Beschreibung

Vorliegende Erfindung betrifft neue 4H-1-Benzopyran-4-on-derivate, Verfahren zu deren Herstellung sowie deren Verwendung als Antiphlogistika, Analgetika, Immunsuppressiva und Antiallergika. Insbesondere betrifft vorliegende Erfindung neue

Verbindungen der Formel I in cis-Form

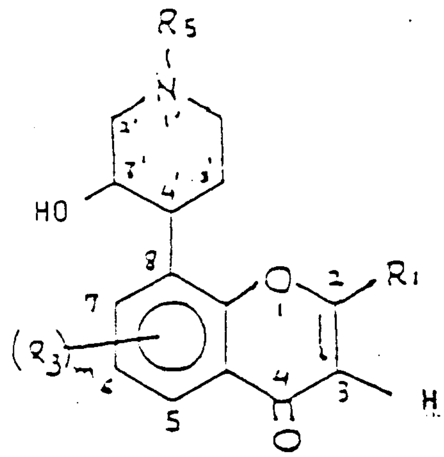

sowie deren pharmakologisch unbedenkliche Säureadditionssalze und optische Isomere, worin bedeuten:

- $R_1$ unsubstituiertes oder mit Halogen, Hydroxy- oder Carboxygruppen substituiertes $C_1$-$C_6$-Alkyl oder Pyridyl oder Phenyl, gegebenenfalls einfach oder mehrfach substituiert mit Halogen, $C_1$-$C_4$-Alkoxy, Nitro, Hydroxy, Carbonyl, Amino, Pyridyl oder Trifluormethyl,
- $R_3$ Hydroxy oder $C_1$-$C_4$-Alkoxy,
- $R_5$ Wasserstoff, $C_1$-$C_6$-Alkyl oder $C_3$-$C_6$-Cycloalkyl-$C_1$-$C_4$-alkyl,
- m eine ganze Zahl zwischen 0 und 3,

mit Ausnahme aller Formen der Verbindung -cis-5,7-Di-hydroxy-2-methyl-8-[4'-(3'-hydroxy-1'-methyl)-piperidinyl]-4H-1-benzopyran-4-on.

Die erfindungsgemäßen Verbindungen besitzen zwei asymmetrische Zentren, eines an der Verknüpfungsstelle des Stickstoffheterocyclus mit dem Benzopyranteil (C-4') und das andere am durch $R_4$ substituierten Kohlenstoffatom (C-3'), so daß zwei optische Isomerenpaare möglich sind. Es versteht sich, daß sämtliche möglichen Stereoisomeren und deren Gemische in der Definition der erfindungsgemäßen Verbindungen inbegriffen sind. Insbesondere sind dabei sowohl die racemischen Formen als auch die isolierten, die angegebene Aktivität besitzenden optischen Isomeren inbegriffen. Die beiden Racemate lassen sich nach physikalischen Methoden wie fraktionierte Kristallisation trennen. Die einzelnen optischen Isomeren sind aus den Racematen nach Standardmethoden wie Salzbildung mit einer optisch aktiven Säure und nachfolgende Kristallisation erhältlich.

In der EP-A2-0 137 193 ist bereits die Verbindung (+)-cis-5,7-Dihydroxy-2-methyl-8-[4'-(3'-hydroxy-1'-methyl)piperidinyl]-4H-1-benzopyrano-4-on, deren Isolierung aus der Pflanze Dysoxylum binectariferum sowie ihre Verwendung als Mittel zur Immunmodulation beschrieben. Diese Verbindung ist daher von der vorliegenden Erfindung ausgenommen.

Als Alkylgruppen für $R_1$ und $R_5$ eignen sich beispielsweise geradkettige oder verzweigte Reste mit bis zu 6 und vorzugsweise bis zu 5 Kohlenstoffatomen, z.B. Methyl-, Ethyl-, Propyl-, Isopropyl-, t-Butyl, Pentyl- oder Isopentylgruppen.

Als substituierte Alkylgruppen für $R_1$ eignen sich beispielsweise Halogenalkyl wie Trifluormethyl, Hydroxyalkyl wie Hydroxyäthyl oder Carboxyalkyl wie Carboxyäthyl.

Die Cycloalkylalkylgruppe $R_5$ ist beispielsweise Cyclopropylmethyl.

Geeignete Beispiele für Salze der erfindungsgemäßen Verbindungen mit anorganischen oder organischen Säuren sind das Hydrochlorid, Hydrobromid, Sulfat, Phosphat, Acetat, Oxalat, Tartrat, Citrat, Maleinat oder Fumarat.

Bevorzugte Verbindungen entsprechen der Formel Ia,

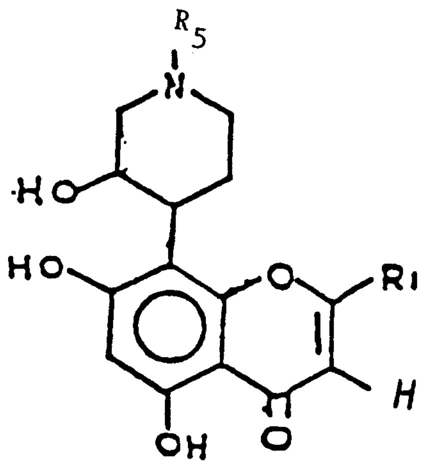

Ia

worin $R_1$, $R_2$ und $R_5$ dieselben Bedeutungen wie oben beschrieben haben und insbesondere bedeuten:

$R_1$ Wasserstoff oder $C_1$-$C_3$-Alkyl

$R_5$ $C_1$-$C_3$-Alkyl oder $C_3$-$C_5$-Cycloalkyl-$C_1$-$C_3$-alkyl.

Besonders bevorzugte erfindungsgemäße Verbindungen sind:

cis-(-)-5,7-Dihydroxy-2-methyl-8-[4'-(1'-cyclopropylmethyl-3'-hydroxy)-piperidinyl]-4H-1-benzopyran-4-on,
cis-(±)-5,7-Dihydroxy-2-äthyl-8-[4'-(3'-hydroxy-1'-methyl)-piperidinyl]-4H-1-benzopyran-4-on und
cis-(±)-5,7-Dihydroxy-2-n-propyl-8-[4'-(3'-hydroxy-1'-methyl)-piperidinyl]-4H-1-benzopyran-4-on.
cis-(+)-5,7-Dihydroxy-2-n-propyl-8-[4'-(3'-hydroxy-1'-methyl)piperidingl-4H-1-benzopyran-4-on.
cis-(-)-5,7-Dihydroxy-2-n-propyl-8-[4'-(3'-hydroxy-1'-methyl)piperidinyl-]-4H-1-benzopyran-4-on.
cis-(±)-2-n-Butyl-5,7-dihydroxy-8-[4'-(3'-hydroxy-1'-methyl)piperidinyl]-4H-1-benzopyran-4-on.
cis-(±)-5,7-Dihydroxy-2-phenyl-8-[4'-(3'-hydroxy-1'-methyl) piperidinyl]-4H-1-benzopyran-4-on.
cis-(-)-5,7-Dihydroxy-2-phenyl-8-[4'-(3'-hydroxy-1'-methyl) piperidinyl]-4H-1-benzopyran-4-on.
cis-(±)-2-(2-Chlorophenyl)-5,7-dihydroxy-8-[4'-(3'-hydroxy-1'-methyl)piperidinyl]-4H-benzopyran-4-on.
cis-(-)-2-(2-Chlorophenyl)-5,7-dihydroxy-8-[4'-(3'-hydroxy-1'-methyl)piperidinyl]-4H-1-benzopyran-4-on.
cis-(±)-2-(4-Aminophenyl)-5,7-dihydroxy-8-[4'-(3'-hydroxy-1'-methyl)piperidinyl]-4H-1-benzopyran-4-on.
cis-(±)-2-(4-Bromophenyl)-5,7-dihydroxy-8-[4'-(3'-hydroxy-1'-methyl)piperidinyl]-4H-1-benzopyran-4-on.
cis-(±)-2-(4-chlorophenyl)-5,7-dihydroxy-8-[4'-(3'-hydroxy-1'-methyl)piperidinyl]-4H-1-benzopyran-4-on.
cis-(±)-2-(2,4-Dichlorophenyl)-5,7-dihydroxy-8-[4'-(3'-hydroxy-1'-methyl)piperidinyl]-4H-1-benzopyran-4-on.
cis-(±)-5,7-Dihydroxy-2-(4-fluorophenyi)-8-[4'-(3'-hydroxy-1'-methyl)piperidinyl]-4H-1-benzopyran-4-on.
cis-(±)-5,7-Dihydroxy-2-(2-fluorphenyl)-8-[4'-(3'-hydroxy-1'-methyl)piperidinyl]-4H-1-benzopyran-4-on.
cis-(±)-5,7-Dihydroxy-2-(2-pyridyl)-8-[4'-(3'-hydroxy-1'-methyl)piperidinyl]-4H-1-benzopyran-4-on.
cis-(±)-5,7-Dihydroxy-2-(4-pyridyl)-8-[4'-(3'-hydroxy-1'-methyl)piperidinyl]-4H-1-benzopyran-4-on.

Neue erfindungsgemäße 4H-1 benzopyran-4-on-derivate sind in den nachfolgenden Tabellen 1 bis 5 aufgeführt, wobei auf die folgenden Formeln Bezug genommen wird:

Formel Ib

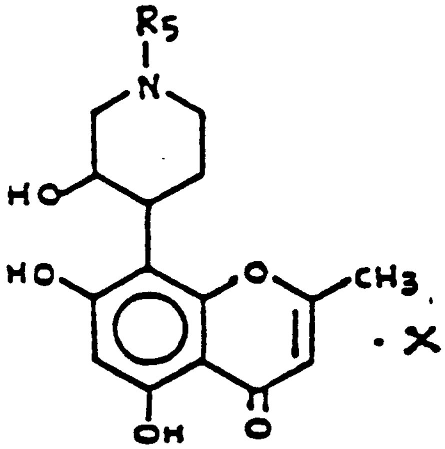

Formel Ic

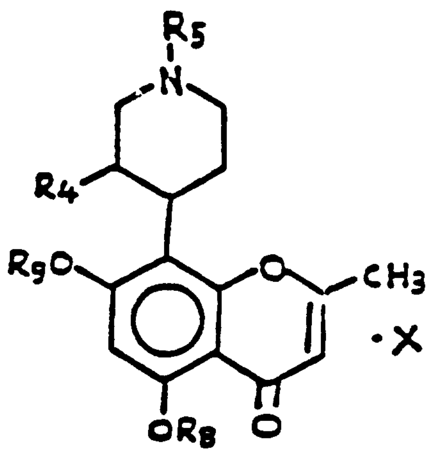

Formel Id

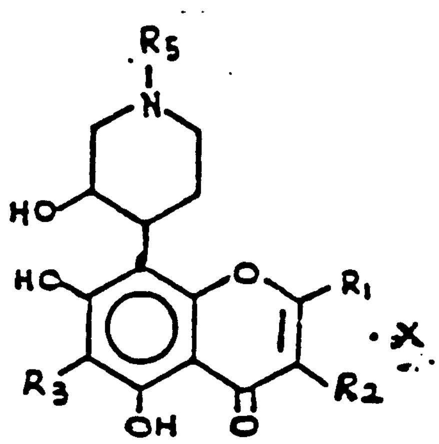

Formel Ie

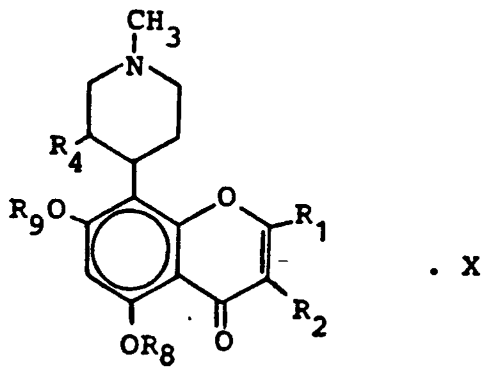

Formel If

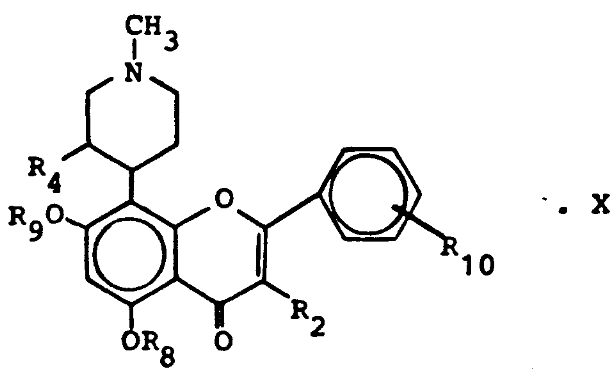

Tabelle 1

Verbindungen der Formel Ib

| $R_5$ | Schmelzpunkt der Base | X | Schmelzpunkt des Salzes |
|---|---|---|---|
| H | >300° | - | - |
| $C_2H_5$ | - | $HCl.H_2O$ | >300° |
| $(CH_2)_2CH(CH_3)_2$ | - | $HCl.3H_2O$ | 265-68° |
| $CH(CH_3)_2$ | >210 | - | - |
| $CH_2-$ 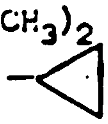 | - | $HCl.H_2O$ | >210 |

Tabelle 2

| Verbindungen der Formel Ic | | | | | |
|---|---|---|---|---|---|
| $R_4$ | $R_5$ | $R_8$ | $R_9$ | X | Schmelzpunkt |
| OH(cis) | $CH_3$ | $CH_3$ | $CH_3$ | - | 236-39° |
| OH(cis) | $C_2H_5$ | $CH_3$ | $CH_3$ | $HCl.\frac{1}{2}\,H_2O$ | 240-42° |
| OH(cis) | $CH_3$ | H | $CH_3$ | HCl | 230-32° |

Tabelle 3

| Verbindungen der Formel Id | | | | | |
|---|---|---|---|---|---|
| $R_1$ | $R_2$ | $R_3$ | $R_5$ | X | Schmelzpunkt |
| $C_2H_5$ | H | H | $CH_3$ | $HCl.1\frac{1}{2}\,H_2O$ | 230-33° |
| n-$C_3H_7$ | H | H | $CH_3$ | $HCl.H_2O$ | 190-92° |

**Tabelle 4**

Verbindungen der Formel Ie

| $R_1$ | $R_2$ | $R_4$ | $R_8$ | $R_9$ | X | Fp. | Opt. Drehung |
|---|---|---|---|---|---|---|---|
| $CH_3$ | H | OH | H | $CH_3$ | HCl | 230 - 232 | (±) |
| $CH_3$ | H | OH | $CH_3$ | $CH_3$ | $2HCl.2H_2O$ | 236 - 239 | (±) |
| $C_2H_5$ | H | OH | H | H | HCl, $1.5H_2O$ | 230 - 233 | (±) |
| $C_2H_5$ | H | OH | $CH_3$ | $CH_3$ | HCl, $1.5H_2O$ | 240 - 242 | (±) |
| $n-C_3H_7$ | H | OH | H | H | HCl | 190 - 192 | (±) |
| $n-C_3H_7$ | H | OH | H | H | HCl, $0.5H_2O$ | 197 - 200 | (+) |
| $n-C_3H_7$ | H | OH | H | H | HCl, $0.5H_2O$ | 198 - 201 | (-) |
| $n-C_4H_9$ | H | OH | H | H | HCl, $H_2O$ | 157 - 159 | (±) |

Tabelle 4 (Forts.)

| $R_1$ | $R_2$ | $R_4$ | $R_8$ | $R_9$ | X | Fp.°C | Opt. Drehung |
|---|---|---|---|---|---|---|---|
| $CF_3$ | H | OH | H | H | 0.5HCl, $2H_2O$ | 160 - 162 | (±) |
| $CF_3$ | H | OH | $CH_3$ | $CH_3$ | HCl | 164 - 65 | (±) |
| 2-Pyridyl | H | OH | H | H | HCl, $0.5H_2O$ | 229° | (±) |
| 3-Pyridyl | H | OH | H | H | 2HCl, $2H_2O$ | 278 - 280 | (±) |
| 4-Pyridyl | H | OH | H | H | 2HCl, $1.5H_2O$ | 236 - 238 | (±) |

**Tabelle 5**

Verbindungen der Formel If

| $R_{10}$ | $R_8$ | $R_9$ | X | Fp. | Opt. Drehung |
|---|---|---|---|---|---|
| H | H | H | HCl, $2H_2O$ | 273 - 275° | (±) |
| 4-$NO_2$ | H | H | HCl, $3H_2O$ | 249° (d) | (±) |
| 4-$NO_2$ | $CH_3$ | $CH_3$ | HCl, $2H_2O$ | 257 - 260° (d) | (±) |
| 2-Cl | H | H | HCl, $H_2O$ | 198 - 200° | (±) |
| 2-Cl | $CH_3$ | $CH_3$ | 1.5HCl, $H_2O$ | 190 - 191° | (±) |
| 4-$NH_2$ | H | H | 2HCl, $2H_2O$ | 240 - 242 | (±) |
| 3,5-Dimethoxy | $CH_3$ | $CH_3$ | 2HCl, $2H_2O$ | 180 - 182° | (±) |
| 4-Br | H | H | HCl, $2H_2O$ | 215° | (±) |
| 4-Cl | H | H | HCl, $1.5H_2O$ | 225° | (±) |
| 2,4-Dichloro | H | H | HCl, $2.5H_2O$ | 165 - 166 | (±) |
| 4-F | H | H | HCl, $H_2O$ | 285 - 287° | (±) |
| 2-F | H | H | HCl, $2H_2O$ | 263 - 265 | (±) |
| 3,5-Dihydroxy | H | H | HCl, $3H_2O$ | 300 - 302 | (±) |
| 3-Cl | H | H | HCl, $2H_2O$ | 288 - 290 | (±) |
| 2-Cl | H | H | HCl, $2H_2O$ | 190 - 192 | (+) |

Tabelle 5 (Fortsetzung)

| $R_{10}$ | $R_8$ | $R_9$ | X | Fp. | Opt. Drehung |
|---|---|---|---|---|---|
| H | H | H | HCl, | 269 - 271 | (+) |
| 3-Br | H | H | HCl, $2H_2O$ | 285° | (±) |
| 2,5-Dichloro | H | H | HCl, $H_2O$ | 251 - 252 | (±) |
| 2-Cl | H | H | HCl, $1.5H_2O$ | 190 - 194 | (-) |
| H | H | H | HCl, $0.5H_2O$ | 266 - .69 | (-) |

Gegenstand der vorliegenden Erfindung ist auch ein Verfahren zur Herstellung von Verbindungen der allgemeinen Formel I, welches die in dem Schema auf der beigefügten Figur 1 skizzierten Stufen umfaßt. Gewünschtenfalls kann man weitere erfindungsgemäße Chromonderivate durch Behandlung von Verbindungen der Formel I' in Figur 1 nach bekannten Methoden erhalten. Das in Figur 1 abgebildete allgemeine Schema wird durch die auf Figur 2 abgebildete Reaktionsfolge mehr im einzelnen erläutert und beschrieben, das sich auf die Herstellung einer der bevorzugten erfindungsgemäßen Verbindung bezieht; dabei versteht es sich daß der Umfang der Erfindung dadurch nicht eingeschränkt wird.

Die Herstellung der Verbindung der Formel VIII mit n = 1 ist dem Fachmann bekannt [S.M. McClavain und R.S. Berger, J. Am. Chem. Soc., 77, 2848 (1955); A. Ziering, L. Berger, S.D. Heineman und J. Lec., J. Org. Chem., 12, 894 (1947)]. Dort sind zwei Methoden beschrieben. Bei der ersten rührt man 1,3,5,-Trimethoxybenzol mit n-Butyllithium bei tiefen Temperaturen, vorzugsweise zwischen -60 und -90 °C, in inerten Lösungsmitteln wie Kohlenwasserstoffen, z.B. Pentan oder Hexan, oder Ätherlösungsmitteln, z.B. Diäthyläther oder Tetrahydrofuran, zur Herstellung des Lithiosalzes, das beim Rühren mit 1-Methyl-4-piperidon und nachfolgendem Ansäuern das Tetrahydropyridinderivat ergibt. Bei der zweiten und besonders bevorzugten Methode rührt man 1,3,5-Trimethoxybenzol unter sauren Bedingungen mit 1-Methyl-4-piperidon in Lösungsmitteln wie Wasser, Essigsäure, alkoholischen Lösungsmitteln oder einem geeigneten Gemisch daraus, wobei Eisessig besonders bevorzugt ist.

Mit Bezug auf das Schema in Figur 2 wird das Tetrahydropyridinderivat der abgebildeten Formel VIIIA (d.h. Formel VIII mit n = 1) unter Verwendung von sich durch Zusatz von $BF_3$-Ätherat zu einer Aufschlämmung von Natriumborhydrid in Diäthylenglykoldimethyläther unter wasserfreien Bedingungen und in einer durch laufendes Durchleiten von Stickstoff oder Argon aufrechterhaltenen inerten Atmosphäre direkt bildendem Diboran hydroboriert. Die Reaktionstemperatur hält man zwischen 20 und 90 °C, doch wird ein Temperaturbereich von 50-60 °C bevorzugt. Der entstandene Organoborankomplex wird zunächst mit Salzsäure behandelt und dann durch Zusatz von Alkali und Wasserstoffperoxyd oxydiert. Die so erhaltene Verbindung ist ein trans-Alkohol der Formel IXA und wird durch Oxydation und nachfolgende Reduktion in den cis-Alkohol der Formel XIA umgewandelt. Die Oxydation des trans-Alkohols der Formel IXA erfolgt mittels einer Reagenzienkombination, nämlich Säurechloriden, wobei Oxalkylchlorid bevorzugt ist, Dimethylsulfoxid und Triäthylamin und ist dem Fachmann als Oxydation nach Swern bekannt. Das durch Oxydation der Verbindung der Formel IXA gebildete Keton der Formel XA wird mittels Hydridreagenzien, vorzugsweise Diboran, Lithiumborhydrid oder Natriumborhydrid, reduziert. Eine ganze Reihe von Lösungsmitteln ist mit Natriumborhydrid verträglich, doch werden protonenhaltige Lösungsmittel wie Methanol, Äthanol und Isopropanol bevorzugt. Durch Einhaltung einer höheren Reaktionstemperatur kann man das cis-Isomer stereoselektiv erhalten.

Das cis-Isomere kann weiterhin gewonnen werden durch fraktionierte Kristallisation seiner Säureadditionssalze, die gebildet werden mit optisch aktiven Säuren wie z. B. (-)- und/oder (+)-Dibenzoylweinsäure. Das cis-Isomere kann gegebenenfalls auch verestert werden mit einer optisch aktiven Säure wie (-)-Menthyloxyessigsäure und die entstehenden diastereomeren Ester können anschließend durch konventionelle Methoden wie fraktionierte Kristallisation oder Chromatograpie getrennt werden.

Die cis-Hydroxyverbindung der Formel XIA wird mit Essigsäureanhydrid und sauren Katalysatoren wie Aluminiumchlorid, Bortrifluoridätherat und Zinn(IV)-chlorid acetyliert, wobei als Reagenz Bortrifluoridätherat besonders bevorzugt ist. Bei Anwendung eines großen Überschusses an Bortrifluoridätherat erfolgt auch gleichzeitig eine Entmethylierung, und nur die gewünschte Methoxylgruppe wird regiospezifisch demethyliert, wobei eine Verbindung der Formel XIIA erhalten wird, worin R den Rest $-COCH_3$ darstellt. Die Hydrolyse dieser Verbindung mit einem Alkalimetallhydroxid führt zu Verbindungen der Formel XIIA mit R = H. Die Verbindung der Formel XIIA wird dann nach an sich bekannten Methoden, von denen zwei hier beschrieben werden, in das Chromon umgewandelt. Bei der ersten Methode wird die Verbindung der Formel XIIA mit R = H bei Raumtemperatur und in inerten Lösungsmitteln wie Äther, Tetrahydrofuran, Dioxan oder Kohlenwasserstofflösungsmitteln wie Hexan mit Essigester und Alkalimetall oder NaH, bevorzugt Natriummetall gerührt; ist der Ester eine niedrigsiedende Flüssigkeit wie im vorliegenden Beispiel, so ist es auch als Lösungsmittel verwendbar. Die Reaktion ist normalerweise nach ein bis zehn Stunden beendet und ergibt das Diketon der Formel XIIIA mit R = H, das beim Rühren mit Mineralsäuren wie Salzsäure oder Schwefelsäure zum Chromon der Formel IA zyklisiert wird. Bei der zweiten Methode wird eine Verbindung der Formel XII A mit R = Ac verestert mit einer geeigneten Säure, z. B. Benzoesäure, und der erhaltene Ester mit einer Base, wie z. B. Alkalihydroxid, in einem inerten Lösungsmittel wie z. B. THF, Dioxan oder Pyridin gerührt, wobei das Chromon der Formel XIII A mit R = Ac und $R_1$ = Phenyl gebildet wird. Verbindungen der Formel Ia mit R = H und $R_1$ = $CH_3$ werden mit Pyridinhydrochlorid entmethoxyliert, um die Hydroxyverbindung der abgebildeten Formel IB zu erhalten. Unter Verwendung der entsprechenden Ester anstelle von Essigester lassen sich verschiedene 2-substituierte Chromone herstellen.

Das Dimethoxychromon der Formel IA kann außer mit $AlCl_3$, $BBr_3$ oder HBr/Essigsäure auch mittels anderer saurer Reagenzien entmethoxyliert werden. Die Entmethoxylierung erfolgt durch Erhitzen der Dimethoxychromonderivate mit Pyridinhydrochlorid für einen Zeitraum von 2 bis 10 Stunden auf 180 °C. In manchen Fällen kann die Zugabe von hochsiedenden Aminen zum Pyridinhydrochlorid vorteilhaft sein.

Das Syntheseschema nach Figur 2 läßt sich zur Herstellung von Verbindungen der Formel I mit $R_5$ = H, Alkyl (von Methyl verschieden), Cycloalkyl, Aralkyl und Aryl anwenden. Verbindungen der Formel I, worin $R_5$ dieselbe Bedeutung wie oben hat, lassen sich auch aus den entsprechenden N-Methylverbindungen, d.h. $R_5$ = $CH_3$ (Verbindungen der Formel IB) nach einer der bekannten Methoden herstellen. Eine typische Arbeitsweise ist aus dem Schema der Figur 3 ersichtlich, wo eine Verbindung der Formel IB mit $R_5$ = $CH_3$ nach Schutz der Hydroxylgruppen mit Bromcyan behandelt und danach sauer oder alkalisch zu Verbindungen mit $R_5$ = H (Verbindung der Formel XIV) hydrolysiert wird. Diese Verbindung ergibt bei Behandlung mit geeigneten elektrophilen Reagenzien wie Halogeniden, Säurechloriden, Tosylaten oder Enonen Verbindungen mit $R_5$ = Alkyl, Cycloalkyl, Aralkyl oder Aryl, wobei die Verbindung der abgebildeten Formel XVII ein spezielles Beispiel darstellt. Gemäß Figur 3 wird 5,7-Dihydroxy-2-methyl-8-[4'-(1'-cyclopropylmethyl-3'-hydroxy)-piperidinyl]-4H-1-benzopyran-4-on der abgebildeten Formel XVII durch Peracetylierung von 5,7-Dihydroxy-2-methyl-8-[4'-(3'-hydroxy-1'-methyl)-piperidinyl]-4H-1-benzopyran-4-on der Formel IB mit Essigsäureanhydrid und Natriumacetat bei 80-90 °C hergestellt. Das peracetylierte Produkt der Formel XIV wird mit Bromcyan in Chloroform verrührt und ergibt in Gegenwart von Kaliumcarbonat 5,7-Diacetyl-2-methyl-8-[4'-(3'-acetoxy-1'-cyan)-piperidinyl]-4H-1-benzopyran-4-on der Formel XV, das bei 5 Stunden langem Erhitzen mit 2n-Salzsäure auf 110 °C das hydrolysierte und N-entmethylierte Produkt 5,7-Dihydroxy-2-methyl-8-[4'-(3'-hydroxy)-piperidinyl]-4H-1-benzopyran-4-on der Formel XVI liefert. Beim Erhitzen mit Cyclopropylmethylchlorid in Isobutylalkohol erhält man daraus das N-Cyclopropylmethylderivat der abgebildeten Formel XVII.

Verbindungen der Formel I, worin $R_2$ Dialkylaminomethyl bedeutet, werden dadurch hergestellt, daß man das entsprechende Chromon mit $R_2$ = H mit einem sekundären Aminhydrochlorid und Paraformaldehyd in Dioxan oder alkoholischen Lösungsmitteln zum Rückfluß erhitzt. Verbindungen der Formel I mit $R_2$ = $NO_2$ werden zweckmäßig durch Verrühren des entsprechenden Chromons mit $R_2$ = H mit Essigsäure und konzentrierter Salpetersäure hergestellt. Verbindungen der Formel I mit $R_2$ = $NH_2$ erhält man aus den entsprechenden Nitroderivaten durch Hydrierung über 10%igem Pd/C.

Verbindungen der Formel I, worin eine der $R_3$-Gruppen für Brom steht, stellt man durch Verrühren der entsprechenden Chromone mit $R_3$ = H mit N-Bromsuccinimid in Dimethylformamid her.

Ein weiteres Merkmal der Erfindung ist, daß die durch die Formel I dargestellten erfindungsgemäßen Verbindungen pharmakologische Eigenschaften besitzen. Insbesondere zeigen sie eine entzündungshemmende und immunomodulierende Wirkung an Laboratoriumstieren. Diese Eigenschaften werden durch die Ergebnisse der nachfolgenden pharmakologischen Prüfungen belegt, die zur Beurteilung der erfindungsgemäßen Verbindungen und ihrer Salze durchgeführt wurden.

Systemische entzündungshemmende Wirkung auf Carrageenininduziertes Pfotenödem an der Ratte

Männliche Charles Foster-Ratten (120-150 g) wurden 18 Stunden lang nüchtern gesetzt, mit Wasser ad libitum. Die in destilliertem Wasser gelöste Testverbindung wurde oral verabreicht. Die Kontrollgruppe erhielt destilliertes Wasser. 0,05 ml 0,5%ige Carrageeninsuspension wurde subkutan in die Sohlengegend der linken Hinterpfote injiziert. Das Pfotenvolumen wurde unter Verwendung eines Differentialvolumenmeßgeräts nach Maclab von der Carrageenininjektion sowie 3 und 6 Stunden danach bestimmt. Die prozentuale Pfotenvolumenabnahme wurde nach folgender Gleichung berechnet:

$$\frac{\text{Mittleres Ödemvolumen in der Trägerkontrolle} - \text{Mittleres Ödemvolumen in der Testgruppe}}{\text{Mittleres Ödemvolumen in der Trägerkontrolle}} \times 100 = \text{\% Pfotenvolumenabnahme}$$

Der $ED_{50}$-Wert wurde aus der Dosis/Wirkungskurve berechnet. Pro Gruppe wurden je sechs Tiere eingesetzt.

Die Ergebnisse mit repräsentativen erfindungsgemäßen Verbindungen und deren Salzen sind in Tabelle 4 angeführt, wobei die Substituenten sich auf die folgende Formel Ia beziehen:

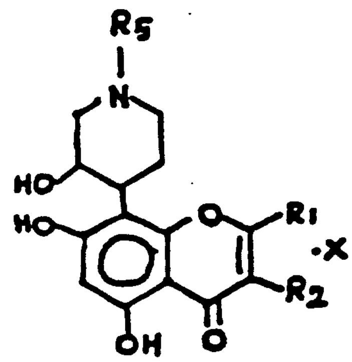

Tabelle 6

Verbindungen der Formel Ia

| cis | VERBINDUNG | | | | |
| --- | --- | --- | --- | --- | --- |
| | $R_1$ | $R_2$ | $R_5$ | X | $ED_{50}$ mg/kg p.o. |
| (−) | $CH_3$ | H | $-CH_2-$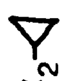 | $HCl.H_2O$ | 10,0 |
| (±) | $C_2H_5$ | H | $CH_3$ | $HCl.1,5\ H_2O$ | 10,5 |
| (±) | $n-C_3H_7$ | H | $CH_3$ | $HCl.H_2O$ | 6,8 |
| (−) | $n-C_3H_7$ | H | H | $HCl,\ 5H_2O$ | 5,8 |
| (±) | Phenyl | H | H | $HCl,\ 2H_2O$ | 5,7 |

Tabelle 6 (Fortsetzung)

| VERBINDUNG | | | | | |
|---|---|---|---|---|---|
| cis | $R_1$ | $R_2$ | $R_5$ | X | $ED_{50}$ mg/kg p.o. |
| (±) | 2-Chlorphenyl | H | H | HCl, $H_2O$ | 2,7 |
| (±) | 4-Aminophenyl | H | H | 2HCl, $2H_2O$ | 7,4 |
| (±) | 4-Chlorphenyl | H | H | HCl, $1{,}5H_2O$ | 7,0 |
| (±) | 2,4-Dichlorphenyl | H | H | HCl, $2{,}5H_2O$ | 5,7 |
| (±) | 4-Fluorphenyl | H | H | HCl, $H_2O$ | 7,4 |
| (±) | 2-Fluorphenyl | H | H | HCl, $2H_2O$ | 7,6 |
| (±) | 2-Pyridyl | H | H | HCl, $5H_2O$ | 5,7 |
| (-) | 2-Chlorphenyl | H | H | HCl, $2H_2O$ | 2,4 |
| (-) | Phenyl | H | H | HCl, $5H_2O$ | 1,3 |

Umgekehrte passive Arthus-Reaktion (RPA) an der Ratte

Charles Foster-Ratten beider Geschlechter von 150-180 g Gewicht wurden in Gruppen von je sechs Tieren sortiert. 24 Stunden vor der Auslösung der RPA wurden die Ratten von der Mittelrückengegend geschoren und über Nacht nüchtern gesetzt. Die Testverbindungen wurden oral eine Stunde vor der Induzierung der Arthus-Reaktion verabreicht. Die RPA-Reaktion wurde durch intradermale Injektion von 0,1 ml entsprechend verdünntem Kaninchen-anti-BSA-Serum induziert. Sofort nach den intradermalen Injektionen erhielten die Ratten intravenös je 0,5 ml 0,4%iges Rinderserumalbumin. Vier Stunden nach der intradermalen Exposition wurden die Tiergruppen jeweils durch Halsbruch getötet. Die gesamte Dicke der

Haut wurde vom Rücken jedes Tiers entfernt, und eine Scheibe von 12 mm Durchmesser wurde mit einer Metallstanze an der Stelle der Antiseruminjektion ausgestanzt. Das Naßgewicht der Hautstelle wurde jeweils so bald wie möglich bestimmt. Das durch die RPA verursachte Ödem wurde als die Differenz (in mg ausgedrückt) zwischen dem Naßgewicht der mit Antikörper injizierten Stelle und der mit normalem Kaninchenserum injizierten Stelle gemessen.

Die Ergebnisse sind als prozentuale Hemmung bzw. Potenzierung des Ödems durch die Verbindung gegenüber dem in den unbehandelten Kontrolltieren induzierten Ödem ausgedrückt.

Die Ergebnisse mit repräsentativen erfindungsgemäßen Verbindungen und deren Salzen sind in Tabelle 7 angeführt.

**Tabelle 7**

**Wirkung auf die umgekehrte passive Arthus-Reaktion (RPA) an der Ratte**

Verbindung der Formel Ia

| VERBINDUNGEN | | | | | DOSIS | % Hemmung |
|---|---|---|---|---|---|---|
| cis | $R_1$ | $R_2$ | $R_5$ | X | mg/kg p.o. | |
| (-) | $CH_3$ | H | $CH_2$-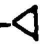 | $HCl.H_2O$ | 1,25 | - |
| | | | | | 2,5 | 23,0 |
| | | | | | 5,0 | 49,7 |
| | | | | | 10,0 | - |
| | | | | | 20,0 | 74,0 |
| | | | | | 2,5 | 48,78 |
| | | | | | 5,0 | 57,15 |
| | | | | | 10,0 | 50,37 |
| | | | | | 20,0 | 43,24 |
| | | | | | 2,5 | 39,11 |
| | | | | | 5,0 | 40,13 |
| | | | | | 10.0 | 45,17 |
| | | | | | 20,0 | 64,73 |
| | | | | | 2,50 | 31,8 |
| | | | | | 5,0 | 29,8 |
| | | | | | 10,0 | 35,8 |
| | | | | | 20,0 | 39,2 |
| (±) | $C_2H_5$ | H | $CH_3$ | $HCl.1\frac{1}{2}H_2O$ | 1,25 | 16,0 |
| | | | | | 2,5 | 34,31 |
| | | | | | 5,0 | 41,65 |
| | | | | | 10,0 | 43,60 |
| | | | | | 20,0 | 77,10 |

Tabelle 7 (Fortsetzung)

| VERBINDUNGEN | | | | | DOSIS | % Hemmung |
|---|---|---|---|---|---|---|
| cis | $R_1$ | $R_2$ | $R_5$ | X | mg/kg p.o. | |
| (±) | n-$C_3H_7$ | H | $CH_3$ | $HCl \cdot H_2O$ | 1,25 | 26,0 |
| | | | | | 2,50 | 32,0 |
| | | | | | 5,0 | 44,0 |
| | | | | | 10,0 | 55,0 |
| | | | | | 20,0 | 65,0 |
| (-) | n-$C_3H_7$ | H | $CH_3$ | $HCl \cdot H_2O$ | 1,25 | -- |
| | | | | | 2,5 | 42,7 |
| | | | | | 5,0 | 41,3 |
| | | | | | 10,0 | 63,7 |
| | | | | | 20,0 | 72,1 |
| (±) | Phenyl | H | $CH_3$ | $HCl \cdot 2H_2O$ | 1,25 | 57,5 |
| | | | | | 2,5 | 55,6 |
| | | | | | 5,0 | 68,1 |
| | | | | | 10,0 | 90,6 |
| | | | | | 20,0 | 95,7 |
| (±) | o-Chlorphenyl | H | $CH_3$ | $HCl \cdot H_2O$ | 1,0 | 37,0 |
| | | | | | 2,0 | 60,0 |
| | | | | | 4,0 | 80,0 |
| (±) | 2,4-Dichlor-phenyl | H | $CH_3$ | $HCl \cdot 2,5\ H_2O$ | 1,25 | 0 |
| | | | | | 2,5 | 41,7 |
| | | | | | 5,0 | 57,2 |
| | | | | | 10,0 | 49,5 |
| | | | | | 20,0 | 76,1 |

Tabelle 7 (Fortsetzung)

| VERBINDUNGEN | | | | | DOSIS | % Hemmung |
|---|---|---|---|---|---|---|
| cis | $R_1$ | $R_2$ | $R_5$ | X | mg/kg p.o. | |
| (±) | p-Fluorphenyl | H | $CH_3$ | $HCl \cdot H_2O$ | 1,25 | 24,4 |
| | | | | | 2,5 | 37,4 |
| | | | | | 5,0 | 61,5 |
| | | | | | 10,0 | 90,0 |
| | | | | | 20,0 | 86,2 |
| (±) | o-Flurophenyl | H | $CH_3$ | $HCl \cdot 2H_2O$ | 1,25 | 0 |
| | | | | | 2,5 | 11,5 |
| | | | | | 5,0 | 52,7 |
| (±) | 2-Pyridyl | H | $CH_3$ | $HCl \cdot 1,5\ H_2O$ | 1,25 | 0 |
| | | | | | 2,5 | 12,0 |
| | | | | | 5,0 | 43,0 |
| | | | | | 10,0 | 90,0 |
| | | | | | 20,0 | 90,0 |
| (-) | 2-Chlorphenyl | H | $CH_3$ | $HCl \cdot 1,5\ H_2O$ | 1,25 | 34,0 |
| | | | | | 2,5 | 48,8 |
| | | | | | 5,0 | 70,0 |
| | | | | | 10,0 | 98,8 |
| | | | | | 20,0 | -- |
| (-) | Phenyl | H | $CH_3$ | $HCl \cdot \frac{1}{2}H_2O$ | 1,25 | -- |
| | | | | | 2,5 | 26,2 |
| | | | | | 5,0 | 64,5 |
| | | | | | 10,0 | 92,2 |
| | | | | | 20,0 | -- |

Die Erfindung wird durch die nachfolgenden Beispiele erläutert, jedoch nicht eingeschränkt.

Beispiel 1

1-Methyl-4-(2,4,6-trimethoxyphenyl)-1,2,3,6-tetrahydropyridin

Man gibt N-Methylpiperidon (2,8 Mol) unter Rühren zu einer Lösung von Trimethoxybenzol (2,38 Mol) in Eisessig (750 ml), wobei man die Temperatur des Reaktionsgemisches unter 25 °C hält. Nach beendeter Zugabe perlt man Chlorwasserstoff durch das Reaktionsgemisch, erhitzt 3 Stunden lang auf 95-100 °C, engt dann ein und verdünnt den Rückstand mit Wasser. Die wäßrige Lösung wird mit Äther extrahiert, der Äther

abgetrennt und die wäßrige Schicht mit konzentrierter Natronlauge alkalisch gemacht. Der so erhaltene Niederschlag wird abfiltriert, mit Wasser gewaschen und getrocknet. Umkristallisieren aus Petroläther (60°-80°C) ergibt 550 g 1-Methyl-3-(2,4,6-trimethoxyphenyl)-1,2,3,6-tetrahydropyridin vom Schmelzpunkt 118-122°C.

| Analyse: Berechnet für $C_{15}H_{21}NO_3.O,5H_2O$ | | | |
|---|---|---|---|
| | C, 66,17; | H, 8,08; | N, 5,14 % |
| Gefunden: | C, 67,75; | H, 7,56; | N, 5,03 % |

Beispiel 2

(±)trans-3-Hydroxy-4-(2,4,6-trimethoxyphenyl)-1-methylpiperidin

Man tropft eine Lösung von $BF_3$-Ätherat (42 ml) in Diäthylenglykoldimethyläther (42 ml) zu einem gekühlten Gemisch aus 1-Methyl-4-(2,4,6-trimethoxyphenyl)-1,2,3,6-tetrahydropyridin (20 g) und Natriumborhydrid (12 g) in Diäthylenglykoldimethyläther (140 ml). Man erhitzt eine Stunde lang auf 50°C und behandelt das abgekühlte Reaktionsgemisch dann mit Wasser (20 ml) und danach mit konzentrierter HCl (116 ml). Man rührt zwei Stunden lang bei 50-60°C, kühlt ab und macht mit Natronlauge alkalisch. Dann wird Wasserstoffperoxydlösung (30 %, 20 ml) dazugegeben und unter Rühren zwei Stunden lang auf 50-60°C erhitzt. Die Lösung wird abgekühlt und mit Essigester extrahiert. Der Essigesterextrakt wird im Vakuum eingeengt. Den Rückstand säuert man mit 2n-HCl an, extrahiert mit Essigester und trennt die organische Schicht ab. Die wäßrige Schicht wird dann mit Natronlauge alkalisch gemacht und mit Äther extrahiert. Der Ätherextrakt wird mit Sole gewaschen, über Natriumsulfat getrocknet und eingeengt, wobei man einen festen Rückstand erhält, der aus heißem Wasser umkristallisiert wird, was trans-3-Hydroxy-4-(2,4,6-trimethoxyphenyl)-1-methylpiperidin (12 g) ergibt. Ausbeute: 12g; Schmelzpunkt 88°-89°C.

| Analyse: Verbindung als Oxalat, berechnet für $C_{15}H_{23}NO_4.0,5(COOH)_2.1,75H_2O$ | | | |
|---|---|---|---|
| | C, 56,5; | H, 7,5; | N, 4,12 % |
| Gefunden: | C, 56,37; | H, 8,14; | N, 4,84 % |

Beispiel 3

(±)1-Methyl-4-(2,4,6-trimethoxyphenyl)-piperidin-3-on

Zu einer auf -60°C gekühlten Lösung von Oxalkylchlorid (20 ml) in trockenem Methylenchlorid (500 ml) tropft man Dimethylsulfoxyd (35 ml) unter Stickstoff und rührt 5-10 Minuten lang. Dann versetzt man mit einer Lösung von (±)-trans-3-Hydroxy-4-(2,4,6-trimethoxyphenyl)-1-methylpiperidin (62 g) in Methylenchlorid (300 ml), während man die Temperatur des Reaktionsgemisches bei -60°C hält. Nach der Zugabe rührt man 15 Minuten lang und gibt Triäthylamin (155 ml) dazu. Dann läßt man das Reaktionsgemisch sich auf eine Temperatur von -30°C erwärmen, verdünnt mit Wasser und macht mit Natriumcarbonat alkalisch.

Die organische Schicht wird abgetrennt und die wäßrige Schicht mit Essigester extrahiert. Die organischen Schichten werden vereinigt, mit Sole gewaschen, über wasserfreiem $Na_2SO_4$ getrocknet und zu einem festen Rückstand eingeengt, der beim Kristallisieren aus Isopropanol das gewünschte Produkt (47 g) vom Schmelzpunkt 110-112°C ergibt.

| Analyse: Verbindung als Hydrochlorid, berechnet für $C_{15}H_{26}NO_4Cl$ | | | | |
|---|---|---|---|---|
| | C, 51,2; | H, 7,39; | N, 3,98; | Cl, 10,09 % |
| Gefunden: | C, 51,77; | H, 7,16; | N, 3,75; | Cl, 11,45 % |

Beispiel 4

(±)cis-3-Hydroxy-4-(2,4,6-trimethoxyphenyl)-1-methylpiperidin

Man gibt Natriumborhydrid (10 g) unter Rühren zu einer am Rückfluß siedenden Lösung von 1-Methyl-4-(2,4,6-trimethoxyphenyl)-piperidin-3-on in absolutem Äthanol. Dann wird noch eine Stunde weitergerührt und am Rückfluß erhitzt. Beim Abkühlen verdünnt man das Reaktionsgemisch mit Wasser, engt dann zur Entfernung des Äthanols ein und extrahiert mit Chloroform. Der Chloroformextrakt wird mit Wasser gewaschen, über wasserfreiem Natriumsulfat getrocknet und zu einem festen Rückstand eingeengt, der beim Kristallisieren aus Aceton das erwünschte Produkt (29,2 g) vom Schmelzpunkt 124-125 °C ergibt.

| Analyse: Verbindung als HCl-Salz; berechnet für $C_{15}H_{24}NO_4Cl$ | | | | |
|---|---|---|---|---|
| | C, 56,69; | H, 7,55; | N, 4,4; | Cl, 11,18 % |
| Gefunden: | C, 56,78; | H, 7,72; | N, 3,93; | Cl, 11,91 % |

Beispiel 5

(±)cis-3-Hydroxy-4-(3'-acetyl-4',6'-dimethoxy-2'-hydroxy)phenyl-1-methylpiperidin

Man tropft $BF_3$-Ätherat (107,6 ml) zu einer Lösung von cis-3-Hydroxy-4-(2,4,6-trimethoxyphenyl)-1-methylpiperidin (35 g) in Methylenchlorid (500 ml) unter Kühlen im Eisbad. Danach werden 76,2 ml Acetanhydrid tropfenweise hinzugefügt. Dann rührt man das Reaktionsgemisch 24 Stunden lang bei Raumtemperatur, verdünnt mit Wasser, macht mit Natriumcarbonat alkalisch und extrahiert mit Methylenchlorid. Der Extrakt wird eingeengt und der Rückstand (37 g) in Methanol (200 ml) gelöst und 2 Stunden lang mit 5 %iger wäßriger Kalilauge (500 ml) gerührt. Dann engt man im Vakuum ein und extrahiert den Rückstand mit Chloroform. Der nach Einengen des Chloroformextrakts erhaltene Rückstand wird dann durch Chromatographie über Silicagel gereinigt, wobei man cis-3-Hydroxy-4-(3'-acetyl-4',6'-dimethoxy-2'-hydroxy)-phenyl-1-methylpiperidin (28 g) vom Schmelzpunkt 215-218 °C (als HCl-Salz) erhält.

| Analyse: Verbindung als HCl-Salz, berechnet für $C_{16}H_{24}NO_5Cl$ | | | | |
|---|---|---|---|---|
| | C, 55,57; | H, 6,94; | N, 4,05; | Cl, 10,27 % |
| Gefunden: | C, 55,24; | H, 7,04; | N, 3,88; | Cl, 10,40 % |

Beispiel 6:

Allgemeines Verfahren zur Herstellung von cis/trans5,7-Dimethoxy-2-($R_1$)-8-[4'-(3'-hydroxy-1-methyl)-piperidinyl]-4H-1-benzopyran-4-one

Die Lösung von cis/trans-3-Hydroxy-4-(3'-acetyl-4,6-dimethoxy-2'-hydroxy)phenyl-1-methylpiperidin (1 Äquival.) wird mit einem geeigneten Ester (3 Äquival.) und Na-Metall (~ 10 Äquival.) oder Na-Hydrid (~ 5 Äquival.) in trockenem Dioxan oder Dimethylformamid bei Raumtemperatur bzw. bei 70 - 80 °C (siehe Tabelle 8) gerührt. Dann wird vorsichtig Wasser zugegeben und mit Chloroform extrahiert. Die organische Phase wird abgetrennt, etwas eingeengt, mit HCl-Gas gesättigt und anschließend eine Stunde gerührt. Die Lösung wird dann durch Zugabe von $Na_2CO_3$ basisch gemacht und mit Chloroform extrahiert. Der Chloroformextrakt wird über wasserfreiem $Na_2SO_4$ getrocknet, im Vakuum eingeengt und mit Hilfe von Säulenchromatographie (über Silicagel) gereinigt. Anschließend kann eine Dünnschichtchromatographie (5 % Methanol in $CHCl_3$ + 1 Vol.-% $NH_4OH$: Rf-Wert 0,5 - 0,7) durchgeführt werden.

Mit dem angegebenen allgemeinen Verfahren wurden die in der folgenden Tabelle 8 und im Beispiel 6a angeführten Verbindungen hergestellt:

Tabelle 8

Herstellung von cis/trans-Verbindungen der Formel Ie

($R_2$ = H, $R_4$ = OH, $R_8$ und $R_9$ = $CH_3$; freie Base)

| $R_1$ | Ester | Lösungsmittel | Base | Temp. | Fp. °C |
|---|---|---|---|---|---|
| Methyl (±) | Ethylacetat | Ethylacetat | Na | Rückfluß | 236-38 |
| " (+) | " | " | " | " | 228-29 |
| " (-) | " | " | " | " | 228-30 |
| Ethyl (±) | Ethylpropionat | Ethylpropionat | " | 70-80°C | 240-42 (HCl) |
| n-Propyl (±) | Ethylbutyrat | Dioxan | " | " | 196-97 |
| " (+) | " | " | " | " | 202-04 |
| " (-) | " | " | " | " | 202-04 |
| Phenyl (±) | Methylbenzoat | DMF | NaH | RT | 232-34 (HCl) |
| " (-) | " | " | " | " | 225-27 |
| 2-Chlorphenyl (±) | Methyl-2-chlorbenzoat | " | " | " | 190-91 (HCl) |
| " (-) | " | " | " | " | 110 |
| p-Bromphenyl (±) | Methyl-2-brombenzoat | " | " | " | 167-70 |
| p-Chlorphenyl (±) | Methyl-2-chlorbenzoat | " | " | " | |
| 2,4-Dichlorphenyl (±) | Methyl-2,4-dichlorbenzoat | " | " | " | 179-81 (HCl) |
| 2-Fluorphenyl (±) | Methyl-2-fluorbenzoat | " | " | " | |
| 4-Fluorphenyl (±) | Methyl-4-fluorbenzoat | " | " | " | 212-14 |
| 2-Pyridyl (±) | Methylpicolinat | " | " | " | 208-10 |
| 4-Pyridyl (±) | Methylisonicotinat | " | " | | 215-17 |

Beispiel 6a:

cis-5,7-Dimethoxy-2-methyl-8-[4'-(3'-hydroxy-1'-methyl)piperidinyl]-4H-1-benzopyran-4-on

Man erhitzt eine Lösung von cis-3-Hydroxy-4-(3'-acetyl-4', 6'-dimethoxy-2'-hydroxy)-phenyl-1-methylpiperidin (10 g) in Essigester (500 ml) zum Rückfluß und gibt Natrium (7 g) in kleinen Anteilen dazu. Man rührt und erhitzt 2 bis 3 Stunden lang am Rückfluß. Nach dem Abkühlen wird das Gemisch mit Wasser

verdünnt und die organische Schicht abgetrennt. Diese wird dann auf das halbe Volumen eingeengt, mit konzentrierter HCl (10 ml) behandelt und etwa eine Stunde lang gerührt. Dann verdünnt man mit Wasser, macht die wäßrige Schicht mit $Na_2CO_3$ alkalisch und extrahiert mit Chloroform. Der Chloroformextrakt wird über wasserfreiem $Na_2SO_4$ getrocknet, im Vakuum eingeengt und durch Säulenchromatographie über Silicagel gereinigt, wobei man das erwünschte Produkt (8 g) erhält. Umkristallisiert aus Chloroform/Petroläther, Schmelzpunkt 236-238 °C (HCl-Salz).

| Analyse: Verbindung als Dihydrochlorid, berechnet für $C_{18}H_{25}NO_5Cl_2$ | | | |
|---|---|---|---|
| C, 50,46; | H, 6,66; | N, 2,68; | Cl, 15,87 % |

Beispiel 7:

Allgemeines Demethylierungsverfahren zur Herstellung von cis/trans-5,7-Dihydroxy-2-($R_1$)-8-[4'-(3'-hydroxy-1'-methyl)piperidinyl]-4H-1-benzopyran-4-on hydrochlorid:
Dimethoxychromon (1,0 g), Pyridinhydrochlorid (5 - 10 g) und Chinolin (0,5 ml) werden gemischt und 2 - 3 Stunden auf 180 - 190 °C erhitzt. Man läßt die Reaktionsmischung dann abkühlen, gibt Wasser (1 ml) hinzu und macht basisch durch Zugeben von festem Natriumbicarbonat. Das halbfeste Produkt wird gründlich mit 20 % Methanol in Chloroform extrahiert, die organische Phase eingeengt und mit Hilfe von Säulenchromatographie (Silicagel; 15 Vol.-% Methanol in Chloroform unter Zusatz von 1 Vol-% $NH_4OH$ als Element; Rf: 0,4 - 0,7) gereinigt. Durch Behandlung mit ätherischem HCl erhält man das Hydrochlorid-Salz.
Mit dem angegebenen allgemeinen Verfahren werden die in der folgenden Tabelle 9 aufgeführten Verbindungen hergestellt:

## Tabelle 9

(Formel Ie mit $R_2 = R_8 = R_9 = H$, $R_4 = OH$)

| $R_1$ | X | mp°C | $[\alpha]_D^{20}$ |
|---|---|---|---|
| Ethyl | HCl $1.5H_2O$ | 230 - 233 | (±) |
| n-Propyl | HCl, $H_2O$ | 190 - 192 | (±) |
| n-Propyl | HCl, $0.5H_2O$ | 197 - 200 | + 33.01° |
| n-Propyl | HCl, $0.5H_2O$ | 198 - 201 | - 25.91 |
| Phenyl | HCl, $2H_2O$ | 273 - 275 | (±) |
| Phenyl | HCl, $H_2O$ | 266 - 269 | - 50.4° |
| 2-Chlorophenyl | HCl, $H_2O$ | 198 - 200 | (±) |
| 2-Chlorophenyl | HCl, $1.5H_2O$ | 190 - 194 | - 3.4° |
| 4-Bromophenyl | HCl, $2H_2O$ | 215 | (±) |
| 4-Chlorophenyl | HCl, $1.5H_2O$ | 225 | (±) |
| 2,4-Dichlorophenyl | HCl, $2.5H_2O$ | 165 - 166 | (±) |

Tabelle 9 (Fortsetzung)

| $R_1$ | X | mp °C | $[\alpha]_D^{20}$ |
|---|---|---|---|
| 2-Fluorophenyl | HCl, $2H_2O$ | 263 - 265 | (+) |
| 4-Fluorophenyl | HCl, $H_2O$ | 285 - 287 | (+) |
| 4-Methylphenyl | HCl, $1.5H_2O$ | 247 - 249 | (+) |
| 2-Pyridyl | HCl, $1.5H_2O$ | 229 | (+) |
| 4-Pyridyl | 2HCl, $2H_2O$ | 278 - 280 | (+) |

Beispiel 8

Spaltung von (±)-cis-3-Hydroxy-1-methyl-4-(2,4,6-trimethoxyphenyl)-piperidin

Man löst die racemische cis-3-Hydroxyverbindung (90 g) in Methanol (300 ml), versetzt mit (-)-Dibenzoylweinsäure (126,4 g) in Methanol (200 ml) und erhitzt zum Sieden. Dann gibt man langsam Diisopropyläther (ca. 500 ml) dazu und läßt die klare Lösung abkühlen. Dabei kristallisiert das Tartratsalz langsam aus. Dieses wird abfiltriert und fünfmal aus Methanol/Diisopropyläther umkristallisiert, $[\alpha]_D^{20}$ = + 48,3° (MeOH). Das Tartratsalz (43 g) wird in Wasser (200 ml) suspendiert, mit Salzsäure (2n, 100 ml)

versetzt und gerührt. Das Reaktionsgemisch wird mit Essigester extrahiert (5 mal je 100 ml). Die Weinsäure wird aus dem Essigesterextrakt zurückgewonnen. Die wäßrige Schicht wird mit Natriumcarbonat alkalisch gemacht und mit Chloroform extrahiert. Der Chloroformextrakt wird über wasserfreiem Natriumsulfat getrocknet und eingeengt, wobei man die (+)-3-Hydroxyverbindung, 17,7 g, Schmelzpunkt 109-111 °C, $[\alpha]_D^{20}$ = + 53,81° (Methanol) erhält.

Die Filtrate von den Tartratkristallisationen werden vereinigt und die freie Base wie oben beschrieben zurückgewonnen. Man löst die freie Base (20 g) in Methanol (110 ml), versetzt mit (+)-Dibenzoylweinsäure (29 g) und erhitzt die Lösung zum Sieden. Dann gibt man langsam Diisopropyläther (110 ml) dazu. Beim Stehen bei Raumtemperatur kristallisiert das Tartrat aus. Es wird abfiltriert und dreimal aus Methanol/Diisopropyläthergemisch umkristallisiert. Ausbeute: 20,2 g, $[\alpha]_D^{20}$ = -49° (MeOH). Die freie Base wird wie oben beschrieben isoliert, Ausbeute: 8,2 g Schmelzpunkt 109-111 °C, $[\alpha]_D^{20}$ = -54,13° (Methanol).

Optisch reine Isomere wurden aus optisch reinen (±)- oder (-)-cis-3-hydroxy-4-(3-Acetyl-4,6-dimethoxy-2-hydroxy)-phenyl-1-methyl-piperidin wie in den folgenden Beispielen 9 und 10 hergestellt:

Beispiel 9

(-)-cis-3-Hydroxy-4-(3'-acetyl-4',6'-dimethoxy-2'-hydroxy)-phenyl-1-methylpiperidin

Man behandelt (-)-cis-3-Hydroxy-4-(2',4',6'-trimethoxyphenyl)-1-methylpiperidin auf die gleiche Weise wie in Beispiel 5, was (-)-cis-3-Hydroxy-4(3'-acetyl-4',6'-dimethoxy-2'-hydroxy)-phenyl-1-methylpiperidin vom Schmelzpunkt 184-86 °C, $[\alpha]_D^{20}$ = -32,63° (MeOH, c = 0,614), ergibt.

Beispiel 10

(+)-cis-3-Hydroxy-4-(3'-acetyl-4',6'-dimethoxy-2'-hydroxy)-phenyl-1-methylpiperidin

Man behandelt (+)-cis-3-Hydroxy-4-(2',4',6'-trimethoxyphenyl)-1-methylpiperidin auf die gleiche Weise wie in Beispiel 5, was (+)-cis-3-Hydroxy-4-(3'-acetyl-4',6'-dimethoxy-2'-hydroxy)-phenyl-1-methylpiperidin vom Schmelzpunkt 184-85 °C, $[\alpha]_D^{20}$ = +34,47° (MeOH, c = 0,586), ergibt.

Beispiel 11

(-)-cis-5,7-Dimethoxy-2-methyl-8-[4'-(3'-hydroxy-1'-methyl)-piperidinyl]-4H-1-benzopyran-4-on

Man behandelt (-)-cis-3-Hydroxy-4-(3'-acetyl-4',6'-dimethoxy-2'-hydroxy)-phenyl-1-methylpiperidin auf die gleiche Weise wie in Beispiel 6, was (-)-cis-5,7-di-methoxy-2-methyl-8-[4'-(3'-hydroxy-1'-methyl)-piperidinyl]-4H-1-benzopyran-4-on vom Schmelzpunkt 228-30 °C, $[\alpha]_D^{20}$ = -80,59° (MeOH, c = 0,59), liefert.

Beispiel 12

(+)-cis-5,7-Dimethoxy-2-methyl-8-[4'-(3'-hydroxy-1'-methyl)-piperidinyl]-4H-1-benzopyran-4-on

Man behandelt (+)-cis-3-Hydroxy-4-(3'-acetyl-4',6'-dimethoxy-2'-hydroxy)-phenyl-1-methylpiperidin auf die gleiche Weise wie in Beispiel 6, was (+)-cis-5,6-dimethoxy-2-methyl-8-[4'-(3'-hydroxy-1'-methyl)-piperidinyl]-4H-1-benzopyran-4-on vom Schmelzpunkt 228-29 °C, $[\alpha]_D^{20}$ = +84,1° (MeOH, c = 0,618), liefert.

Beispiel 13

(-)-cis-5,7-Dihydroxy-2-methyl-8-[4'-(3'-hydroxy-l'-methyl)-piperidinyl]-4H-l-benzopyran-4-on-hydrochlorid

Man behandelt (-)-cis-5,7-Dimethoxy-2-methyl-8-[4'-(3'-hydroxy-l'-methyl)-piperidinyl]-4H-l-benzopyran-4-on auf die gleiche Weise wie in Beispiel 7, was (-)-cis-5,7-Dihydroxy-2-methyl-8-[4'-(3'-hydroxy-l'-methyl)-piperidinyl]-4H-l-benzopyran-4-on-hydrochlorid vom Schmelzpunkt 242-45 °C, $[\alpha]_D^{20}$ = -27,5° (MeOH, c = 0,653), liefert.

Beispiel 14

(+)-cis-5,7-Dihydroxy-2-methyl-8-[4'-(3'-hydroxy-l'-methyl)-piperidinyl]-4H-l-benzopyran-4-on-hydrochlorid

Man behandelt (+)-cis-5,7-Dimethoxy-2-methyl-8-[4'-(3'-hydroxy-l'-methyl)-piperidinyl]-4H-l-benzopyran-4-on auf die gleiche Weise wie in Beispiel 7, was (+)-cis-5,7-Dihydroxy-2-methyl-8-['-(3'-hydroxy-l'-methyl)-piperidinyl]-4H-l-benzopyran-4-on-hydrochlorid vom Schmelzpunkt 242-44 ° C, $[\alpha]_D^{20}$ = +29,57 ° (MeOH, c = 0,58), liefert.

Beispiel 15

cis-5,7-Dihydroxy-2-äthyl-8-[4'-(3'-hydroxy-l'-methyl)-piperidinyl]-4H-l-benzopyran-4-on-hydrochlorid

Man behandelt cis-3-Hydroxy-4-(3'-acetyl-4',6'-dimethoxy-2'-hydroxy)-phenyl-l-methylpiperidin wie in Beispiel 6 mit Propionsäureäthylester anstelle von Essigester und entmethoxyliert das Produkt wie in Beispiel 7 beschrieben, was cis-5,7-Dihydroxy-2-äthyl-8-[4'-(3'-hydroxy-l'-methyl)-piperidinyl]-4H-l-benzopyran-4-on-hydrochlorid vom Schmelzpunkt 230-33 ° liefert.

| Analyse: Berechnet für $C_{19}H_{25}NO_5.HCl.0,5H_2O$ | | | | |
|---|---|---|---|---|
| | C, 53,3; | H, 6,53; | N, 3,66; | Cl, 9,28% |
| Gefunden: | C, 53,1; | H, 6,51; | N, 3,83; | Cl, 9,45% |

Beispiel 16

cis-5,7-Dihydroxy-2-n-propyl-8-[4'-(3'-hydroxy-l'-methyl)-piperidinyl]-4H-l-benzopyran-4-on-hydrochlorid

Man behandelt cis-3-Hydroxy-4-(3'-acetyl-4',6'-dimethoxy-2'-hydroxy)-phenyl-l-methylpiperidin wie in Beispiel 6 mit Buttersäureäthylester anstelle von Essigester und entmethoxyliert das Produkt wie in Beispiel 7 beschrieben, was cis-5,7-Dihydroxy-2-n-propyl-8-[4'-(3'-hydroxy-l'-methyl)-piperidinyl]-4H-l-benzopyran-4-on-hydrochlorid vom Schmelzpunkt 190-92 ° C liefert.

| Analyse: Berechnet für $C_{20}H_{27}NO_5.HCl.H_2O$ | | | | |
|---|---|---|---|---|
| | C, 55,74; | H, 6,70; | N, 3,61; | Cl, 9,16% |
| Gefunden: | C, 56,25; | H, 6,65; | N, 3,52; | Cl, 9,39% |

Beispiel 17

cis-(-)-5,7-Dihydroxy-2-methyl-8-[4'-(3'-hydroxy)-piperidinyl]-4H-l-benzopyran-4-on

Man erhitzt cis-(-)-5,7-Dihydroxy-2-methyl-8-[4'-(3'-hydroxy-l'-methyl)-piperidinyl]-4H-l-benzopyran-4-on (5 g) mit Essigsäureanhydrid (25 ml) und Natriumacetat (4,5 g) 12 Stunden lang auf 90 ° C. Das Essigsäureanhydrid wird im Hochvakuum abdestilliert und der Rückstand mit Essigester angerührt. Der in Essigester lösliche Anteil wird zur Trockne eingeengt. Den Rückstand löst man in trockenem Chloroform (27 ml), gibt wasserfreies Kaliumcarbonat (5 g) dazu und kühlt auf 0 ° C ab. Bromcyan (6 g) in trockenem Chloroform (25 ml) wird dazugetropft. Nach der Zugabe rührt man das Reaktionsgemisch 4-5 Stunden lang bei 40-50 ° C, filtriert und wäscht das Filtrat mit einer kleinen Menge Sole, trocknet über wasserfreiem Natriumsulfat und engt ein. Der Rückstand wird auf dem Dampfbad 7-8 Stunden lang mit 1n Salzsäure (30 ml) erhitzt. Das Reaktionsgemisch wird durch Zugabe festen Natriumcarbonats alkalisch gemacht und eingeengt. Den Rückstand läßt man durch eine HP-20-Säule laufen und eluiert das Produkt mit 20%igem MeOH in $H_20$. Das Produkt wird aus MeOH/Diisopropyläther kristallisiert, Schmelzpunkt 300 ° C, $[\alpha]_D^{20}$ =-11,38 ° (MeOH, c = 0,9).

| Analyse: Verbindung als Hydrochloridsalz, berechnet für $C_{15}H_{18}NO_5Cl$ | | | | |
|---|---|---|---|---|
| | C, 55,00; | H, 5,53; | N, 4,27; | Cl, 10,81% |
| Gefunden: | C, 54,33; | H, 5,59; | N, 3,93; | Cl, 11,21% |

| Analyse: Berechnet für $C_{20}H_{27}NO_5.HCl.H_2O$ | | | | |
|---|---|---|---|---|
| | C, 55,74; | H, 6,70; | N, 3,61; | Cl, 9,16% |
| Gefunden: | C, 56,25; | H, 6,65; | N, 3,52; | Cl, 9,39% |

Beispiel 18

cis-(-)-5,7-Dihydroxy-2-methyl-8-[4'-(l'-cyclopropylmethyl 3'-hydroxy)-piperidinyl]-4H-l-benzopyran-4-on-hydrochlorid

Man vermischt cis-(-)-5,7-Dihydroxy-2-methyl-8-[4'-(3'-hydroxy)-piperidinyl]-4H-l-benzopyran-4-on (1,0 g), Cyclopropylmethylketon (1,5 ml), Isobutanol (15 ml) und Kaliumcarbonat (3 g) und erhitzt 15 Stunden lang auf 90 °C. Das Reaktionsgemisch wird filtriert und der Rückstand mit Chloroform gewaschen. Das Filtrat wird eingeengt und über Silicagel säulenchromatographiert. Die Verbindung wird mit 6%igem MeOH in Chloroform eluiert. Durch Zusatz von ätherischer HCl stellt man das Hydrochlorid her, Ausbeute 0,7 g, Schmelzpunkt 249-51 °C, $[\alpha]_D^{20}$ = -35,4° (MeOH, c= 0,571).

| Analyse: Berechnet für $C_{19}H_{26}NO_6Cl$ | | | | |
|---|---|---|---|---|
| | C, 57,07; | H, 6,51; | N, 3,75; | Cl, 8,87% |
| Gefunden: | C, 57,18; | H, 6,51; | N, 3,75; | Cl, 9,44% |

**Patentansprüche**

**Patentansprüche für folgende Vertragsstaaten : BE, CH, DE, FR, GB, IT, LI, LU, NL, SE**

**1.** Verbindungen der Formel I in cis-Form

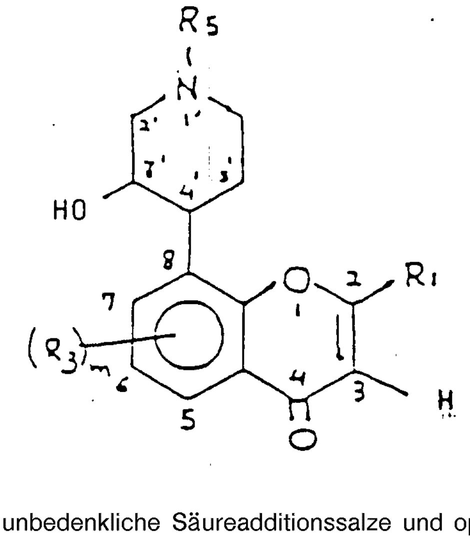

sowie deren pharmakologisch unbedenkliche Säureadditionssalze und optische Isomere, worin bedeuten:

- $R_1$ unsubstituiertes oder mit Halogen, Hydroxy- oder Carboxygruppen substituiertes $C_1$-$C_6$-Alkyl oder Pyridyl oder Phenyl, gegebenenfalls einfach oder mehrfach substituiert mit Halogen, $C_1$-$C_4$-Alkoxy, Nitro, Hydroxy, Carbonyl, Amino, Pyridyl oder Trifluormethyl,
- $R_3$ Hydroxy oder $C_1$-$C_4$-Alkoxy,
- $R_5$ Wasserstoff, $C_1$-$C_6$-Alkyl oder $C_3$-$C_6$-Cycloalkyl-$C_1$-$C_4$-alkyl,

m eine ganze Zahl zwischen 0 und 3,
mit Ausnahme aller Formen der Verbindung -cis-5,7-Di-hydroxy-2-methyl-8-[4'-(3'-hydroxy-1'-methyl)-piperidinyl]-4H-1-benzopyran-4-on.

2. Verbindungen gemäß Anspruch 1, worin $R_1$, und $R_5$ die genannte Bedeutung haben, $R_3$ die Hydroxylgruppe, und m die Zahl 2 bedeuten.

3. Verbindungen gemäß Anspruch 1, worin $R_1$ Wasserstoff oder $C_1$-$C_3$-Alkyl, $R_3$ die Hydroxygruppe, $R_5$ $C_1$-$C_3$-Alkyl und, m die Zahl 2 bedeuten.

4. cis-(±)-2-(2-Chlorphenyl)-5,7-dihydroxy-8-[4'-(3'-hydroxy-1'-methyl)piperidinyl]-4H-1-benzopyran-4-on sowie dessen pharmakologisch unbedenklichen Säureadditionssalze.

5. cis-(-)-2-(2-Chlorphenyl)-5,7-dihydroxy-8-[4'-(3'-hydroxy-1-methyl)piperidinyl]-4H-1-benzopyran-4-on sowie dessen pharmakologisch unbedenklichen Säureadditionssalze.

6. cis-(-)-2-Phenyl-5,7-dihydroxy-8-[4'-(3'-hydroxy-1'-methyl)piperidinyl]-4H-1-benzopyran-4-on sowie dessen pharmakologisch unbedenklichen Säureadditionssalze.

7. cis-(±)-2-Phenyl-5,7-dihydroxy-8-[4'-(3'-hydroxy-1'-methyl)piperidinyl]-4H-1-benzopyran-4-on sowie dessen pharmakologisch unbedenklichen Säureadditionssalzen.

8. cis-(±)2-(p-Fluorpnenyl)-5,7-dihydroxy-8-[4'-(3'-hydroxy-1'-methyl)piperidinyl]-4H-1-benzopyran-4-on sowie dessen pharmakologisch unbedenklichen Säureadditionssalze.

9. cis-(±)-2-(2-pyridyl)-8-[4'-(3'-hydroxy-1'-methyl)-piperidinyl]-4H-benzopyran-4-on sowie dessen pharmakologisch unbedenklichen Säureadditionssalze.

10. Verfahren zur Herstellung von Verbindungen der Formel I gemäß Anspruch 1

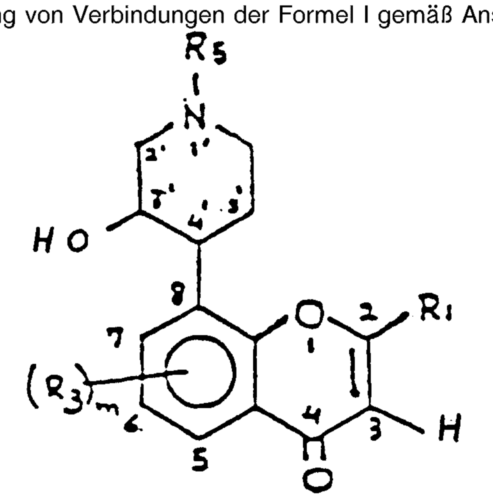

I

worin $R_1$ bis $R_5$ sowie in die in Anspruch 1 genannten Bedeutungen haben
sowie deren pharmakologisch unbedenklichen Säureadditionssalze und optischen Isomeren,
dadurch gekennzeichnet, daß man eine Verbindung der Formel XII

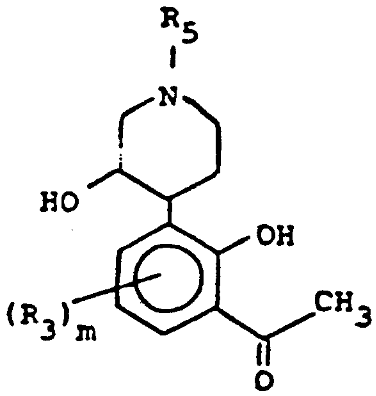

XII

worin $R_3$, $R_5$ und m die genannte Bedeutung haben, mit einem Alkalimetall oder Alkalimetallhydrid und dem Alkylester einer Säure der Formel $R_1$-COOAlkyl, wobei $R_1$ die zur Formel I genannte Bedeutung hat, umsetzt zu einem Diketon der Formel XIII

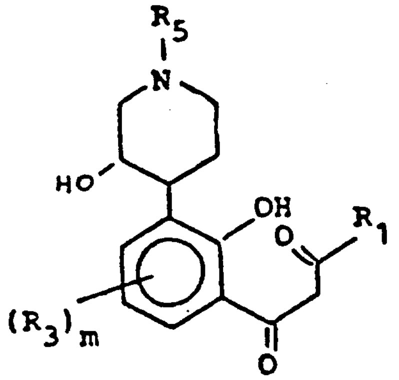

XIII

und die erhaltene Verbindung durch Umsetzen mit einer Mineralsäure zyklisiert zu einer Verbindung der Formel I,
worin $R_1$, $R_3$, $R_5$ und m die angegebene Bedeutung haben, gegebenenfalls eine Verbindung der Formel I demethoxyliert zu einer Verbindung der Formel I, worin $R_3$ Hydroxy bedeutet und m die genannte Bedeutung hat, und gegebenenfalls eine Verbindung der Formel I, worin $R_5$ $CH_3$ bedeutet, nach Schutz der Hydroxylgruppen mit Bromcyan umsetzt und die erhaltene Verbindung sauer oder alkalisch umsetzt zu einer Verbindung der Formel I, worin $R_5$ Wasserstoff bedeutet,
gegebenenfalls einer Verbindung der Formel I, worin $R_5$ Wasserstoff bedeutet, mit geeigneten elektrophilen Reagenzien wie Halogeniden, Säurechloriden, Tosylaten oder Enonen zu Verbindungen der Formel I umsetzt, worin $R_5$ unsubstituiertes oder substituiertes $C_1$-$C_6$-Alkyl, oder $C_3$-$C_6$-Cycloalkyl-$C_1$-$C_4$-alkyl bedeutet,

**11.** Arzneimittel, gekennzeichnet durch einen Gehalt an einer Verbindung der Formel I gemäß Anspruch 1 oder einem pharmakologisch unbedenklichen Säureadditionssalz.

**12.** Verwendung einer Verbindung der Formel I gemäß Anspruch 1 zur Herstellung eines Arzneimittels mit entzündungshemmender und/oder immunmodulierender Wirkung.

**Patentansprüche für folgende Vertragsstaaten : AT, ES, GR**

**1.** Verfahren zur Herstellung von Verbindungen der Formel I in cis-Form

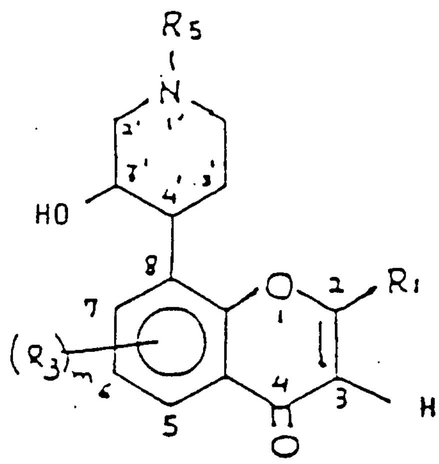

sowie deren pharmakologisch unbedenklichen Säureadditionssalzen und optischen Isomeren, worin bedeuten:

- $R_1$ unsubstituiertes oder mit Halogen, Hydroxy- oder Carboxygruppen substituiertes $C_1$-$C_6$-Alkyl oder Pyridyl oder Phenyl, gegebenenfalls einfach oder mehrfach substituiert mrt Halogen, $C_1$-$C_4$-Alkoxy, Nitro, Hydroxy, Carbonyl, Amino, Pyridyl oder Trifluormethyl,
- $R_3$ Hydroxy oder $C_1$-$C_4$-Alkoxy,
- $R_5$ Wasserstoff, $C_1$-$C_6$-Alkyl oder $C_3$-$C_6$-Cycloalkyl-$C_1$-$C_4$-alkyl,
- m eine ganze Zahl zwischen 0 und 3,

mit Ausnahme aller Formen der Verbindung -cis-5,7-Di-hydroxy-2-methyl-8-[4'-(3'-hydroxy-1'-methyl)-piperidinyl]-4H-1-benzopyran-4-on,

, dadurch gekennzeichnet, daß man eine

Verbindung der Formel XII

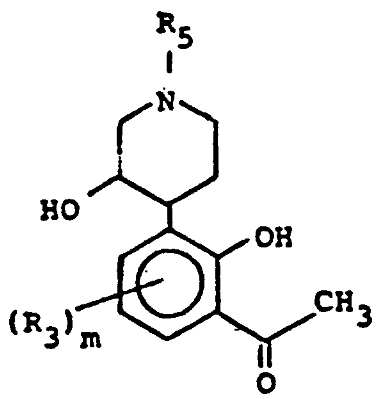

XII

worin $R_3$, $R_5$ und m die genannte Bedeutung haben, mit einem Alkalimetall und dem Alkylester einer Säure der Formel $R_1$-COOAlkyl, wobei $R_1$ die zur Formel I genannte Bedeutung hat, umsetzt zu einem Diketon der Formel XIII

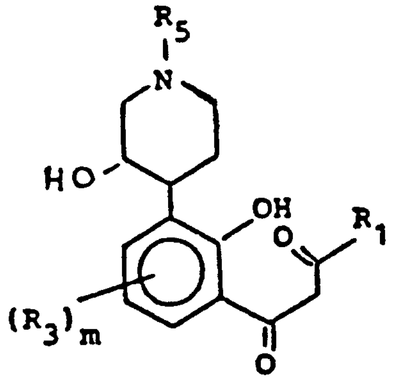

XIII

und die erhaltene Verbindung durch Umsetzen mit einer Mineralsäure zyklisiert zu einer Verbindung der Formel I,
worin $R_1$, $R_3$, $R_5$ und m die angegebene Bedeutung haben, , gegebenenfalls eine Verbindung der Formel I demethoxyliert zu einer Verbindung der Formel I, worin $R_3$ die Hydroxygruppe bedeutet und m die genannte Bedeutung hat,
und gegebenenfalls eine Verbindung der Formel I, worin $R_5$ $CH_3$ bedeutet, nach Schutz der Hydroxylgruppen mit Bromcyan umgesetzt und die erhaltene Verbindung sauer oder alkalisch umgesetzt wird zu einer Verbindung der Formel I, worin $R_5$ Wasserstoff bedeutet,
gegebenenfalls eine Verbindung der Formel I, worin $R_5$ Wasserstoff bedeutet, mit geeigneten elektrophilen Reagenzien wie Halogeniden, Säurechloriden, Tosylaten oder Enonen zu Verbindungen der Formel I umsetzt, worin $R_5$ unsubstituiertes oder substituiertes $C_1$-$C_6$-Alkyl, oder $C_3$-$C_6$-Cycloalkyl-$C_1$-$C_4$-alkyl bedeutet,

**2.** Verfahren gemäß Anspruch 1, dadurch gekennzeichnet, daß $R_1$ und $R_5$ die genannte Bedeutung haben $R_3$ die Hydroxylgruppe sowie m die Zahl 2 bedeuten.

**3.** Verfahren gemäß Anspruch 1, dadurch gekennzeichnet, daß $R_1$ Wasserstoff oder $C_1$-$C_3$-Alkyl, $R_3$ die Hydroxylgruppe, $R_5$ $C_1$-$C_3$-Alkyl, und m die Zahl 1 bedeuten.

**4.** Verfahren gemäß Anspruch 1, dadurch gekennzeichnet, daß cis-(±)-2-(2-Chlorphenyl)-5,7-dihydroxy-8-[4'-(3'-hydroxy-1'-methyl)piperidinyl]-4H-1-benzopyran-4-on oder dessen pharmakologisch unbedenklichen Säureadditionssalze dargestellt werden.

**5.** Verfahren gemäß Anspruch 1, dadurch gekennzeichnet, daß cis-(-)-2-(2-Chlorophenyl)-5,7-dihydroxy-8-[4'-(3-hydroxy-1-methyl)piperidinyl]-4H-1-benzopyran-4-on oder dessen pharmakologisch unbedenklichen Säureadditionssalze hergestellt werden.

**6.** Verfahren gemäß Anspruch 1, dadurch gekennzeichnet, daß cis-(-)-2-Phenyl-5,7-dihydroxy-8-[4'-(3'-hydroxy-1'-methyl)piperidinyl]-4H-1-benzopyran-4-on oder dessen pharmakologisch unbedenklichen Säureadditionssalze hergestellt werden.

**7.** Verfahren gemäß Anspruch 1, dadurch gekennzeichnet, daß cis-(±)-2-Phenyl-5,7-dihydroxy-8-[4'-(3'-hydroxy-1'-methyl)piperidinyl]-4H-1-benzopyran-4-n oder dessen pharmakologisch unbedenklichen Säureadditionssalze hergestellt werden.

**8.** Verfahren gemäß Anspruch 1, dadurch gekennzeichnet, daß cis-(±)-2-(p-Fluorphenyl)-5,7-dihydroxy-8-[4'-(3'-hydroxy-1'-methyl)piperidinyl]-4H-1-benzopyran-4-on oder dessen pharmakologisch unbedenklichen Säureadditionssalze hergestellt werden.

**9.** Verfahren gemäß Anspruch 1, dadurch gekennzeichnet, daß cis-(±)-2-(2-pyridyl)-8-[4'-(3'-hydroxy-1'-methyl)-piperidinyl]-4H-benzopyran-4-on oder dessen pharmakologisch unbedenkliche Säureadditionssalze hergestellt werden.

**10.** Verwendung einer Verbindung der Formel I gemäß Anspruch 1 zur Herstellung eines Arzneimittels mit entzündungshemmendender und/oder immunmodulatorischer Wirkung.

## Claims

## Claims for the following Contracting States: BE, CH, DE, FR, GB, IT, LI, LU, NL, SE

**1.** A compound of the formula I in the cis-form

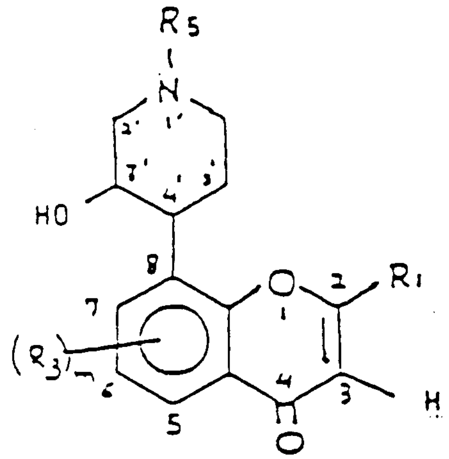

and pharmacologically acceptable acid addition salts and optical isomers thereof, in which:

- $R_1$ is unsubstituted or halogen-, hydroxyl- or carboxyl-substituted $C_1$-$C_6$-alkyl or pyridyl or phenyl which may be mono- or poly-substituted by halogen, $C_1$-$C_4$-alkoxy, nitro, hydroxyl, carbonyl, amino, pyridyl or trifluoromethyl,
- $R_3$ is hydroxyl or $C_1$-$C_4$-alkoxy,
- $R_5$ is hydrogen, $C_1$-$C_6$-alkyl or $C_3$-$C_6$-cycloalkyl-$C_1$-$C_4$-alkyl, and
- m is an integer from 0 to 3,

with the exception of all forms of the compound cis-5,7-dihydroxy-2-methyl-8-[4'-(3'-hydroxy-1'-methyl)-piperidinyl]-4H-1-benzopyran-4-one.

**2.** A compound as claimed in claim 1, wherein $R_1$ and $R_5$ are as defined, $R_3$ is a hydroxyl group and m is the number 2.

**3.** A compound as claimed in claim 1, wherein $R_1$ is hydrogen or $C_1$-$C_3$-alkyl, $R_3$ is a hydroxyl group, $R_5$ is $C_1$-$C_3$-alkyl and m is the number 2.

**4.** cis-(±)-2-(2-Chlorophenyl)-5,7-dihydroxy-8-[4'-(3'-hydroxy-1'-methyl)-piperidinyl]-4H-1-benzopyran-4-one and pharmacologically acceptable acid addition salts thereof.

**5.** cis-(-)-2-(2-Chlorophenyl)-5,7-dihydroxy-8-[4'-(3'-hydroxy-1'-methyl)-piperidinyl]-4H-1-benzopyran-4-one and pharmacologically acceptable acid addition salts thereof.

**6.** cis-(-)-2-Phenyl-5,7-dihydroxy-8-[4'-(3'-hydroxy-1'-methyl)-piperidinyl]-4H-1-benzopyran-4-one and pharmacologically acceptable acid addition salts thereof.

**7.** cis-(±)-2-Phenyl-5,7-dihydroxy-8-[4'-(3'-hydroxy-1'-methyl)-piperidinyl]-4H-1-benzopyran-4-one and pharmacologically acceptable acid addition salts thereof.

**8.** cis-(±)-2-(p-Fluorophenyl)-5,7-dihydroxy-8-[4'-(3'-hydroxy-1'-methyl)-piperidinyl]-4H-1-benzopyran-4-one and pharmacologically acceptable acid addition salts thereof.

**9.** cis-(±)-2-(2-Pyridyl)-8-[4'-(3'-hydroxyl-1'-methyl)-piperidinyl]-4H-benzopyran-4-one and pharmacologically acceptable acid addition salts thereof.

**10.** A process for preparing a compound of the formula I as claimed in claim 1

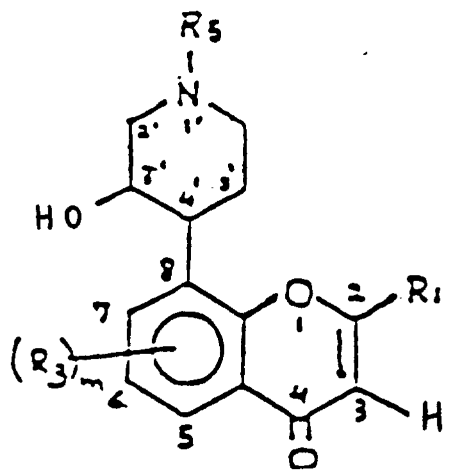

I

in which $R_1$ to $R_5$ and m are as defined in claim 1 and pharmacologically acceptable acid addition salts and optical isomers thereof,
which comprises reacting a compound of the formula XII

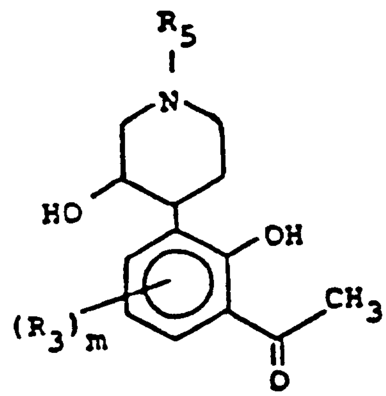

XII

in which $R_3$, $R_5$ and m are as defined, with an alkali metal or alkali metal hydride and the alkyl ester of an acid, of the formula $R_1$-COO-alkyl, $R_1$ being as defined under the formula I, to give a diketone of the formula XIII

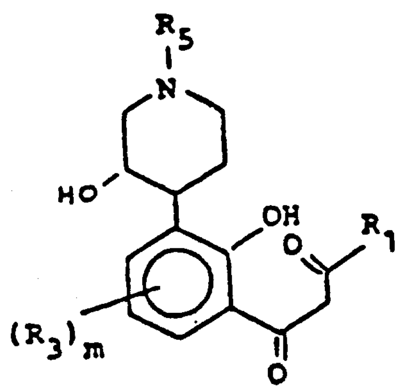

XIII

and cyclizing the resulting compound by reaction with a mineral acid to give a compound of the formula I,
in which $R_1$, $R_3$, $R_5$ and m are as defined, and, if appropriate, demethoxylating a compound of the formula I to give a compound of the formula I in which $R_3$ is hydroxyl and m is as defined, and, if appropriate, reacting a compound of the formula I in which $R_5$ is $CH_3$, after protecting the hydroxyl groups, with cyanogen bromide and reacting the resulting compound under acidic or alkaline conditions to give a compound of the formula I in which $R_5$ is hydrogen,
if appropriate, reacting a compound of the formula I in which $R_5$ is hydrogen with suitable electrophilic

reagents, such as halides, acid chlorides, tosylates and enones, to give a compound of the formula I in which $R_5$ is unsubstituted or substituted $C_1$-$C_6$-alkyl or $C_3$-$C_6$-cycloalkyl-$C_1$-$C_4$-alkyl.

**11.** A medicament, containing a compound of the formula I as claimed in claim 1 or a pharmacologically acceptable acid addition salt thereof.

**12.** The use of a compound of the formula I as claimed in claim 1 for the preparation of a medicament having an anti-inflammatory and/or immunomodulating action.

**Claims for the following Contracting States: AT, ES, GR**

**1.** A process for preparing a compound of the formula I in cis form

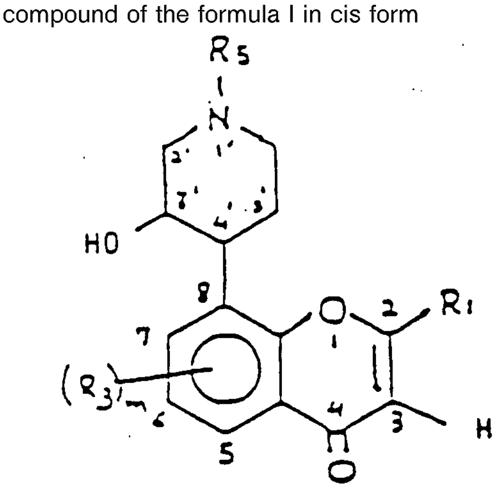

and pharmacologically acceptable acid addition salts and optical isomers thereof, in which

- $R_1$ is unsubstituted or halogen-, hydroxyl- or carboxyl-substituted $C_1$-$C_6$-alkyl or pyridyl or phenyl which may be mono- or poly-substituted by halogen, $C_1$-$C_4$-alkoxy, nitro, hydroxyl, carbonyl, amino, pyridyl or trifluoromethyl,
- $R_3$ is hydroxyl or $C_1$-$C_4$-alkoxy,
- $R_5$ is hydrogen, $C_1$-$C_6$-alkyl or $C_3$-$C_6$-cycloalkyl-$C_1$-$C_4$-alkyl, and
- m is an integer from 0 to 3,

with the exception of all forms of the compound cis-5,7-dihydroxy-2-methyl-8-[4'-(3'-hydroxy-1'-methyl)-piperidinyl]-4H-1-benzopyran-4-one, which comprises reacting a compound of the formula XII

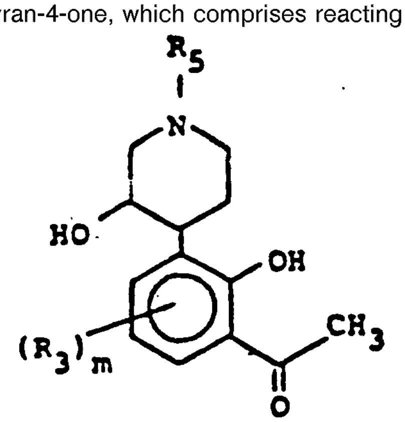

XII

in which $R_3$, $R_5$ and m are as defined, with an alkali metal and the alkyl ester of an acid, of the formula $R_1$-COO-alkyl, $R_1$ being as defined under the formula I, to give a diketone of the formula XIII

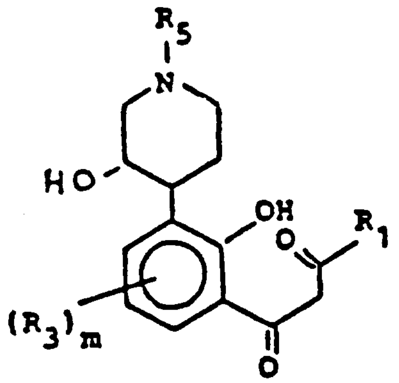

XIII

and cyclizing the resulting compound by reaction with a mineral acid to give a compound of the formula I,

in which $R_1$, $R_3$, $R_5$ and m are as defined, and, if appropriate, demethoxylating a compound of the formula I to give a compound of the formula I in which $R_3$ is hydroxyl and m is as defined, and, if appropriate, reacting a compound of the formula I in which $R_5$ is $CH_3$, after protecting the hydroxyl groups, with cyanogen bromide and reacting the resulting compound under acidic or alkaline conditions to give a compound of the formula I in which $R_5$ is hydrogen,

if appropriate, reacting a compound of the formula I in which $R_5$ is hydrogen with suitable electrophilic reagents, such as halides, acid chlorides, tosylates and enones, to give a compound of the formula I in which $R_5$ is unsubstituted or substituted $C_1$-$C_6$-alkyl or $C_3$-$C_6$-cycloalkyl-$C_1$-$C_4$-alkyl.

**2.** The process as claimed in claim 1, wherein $R_1$ and $R_5$ are as defined, $R_3$ is a hydroxyl group and m is the number 2.

**3.** The process as claimed in claim 1, wherein $R_1$ is hydrogen or $C_1$-$C_3$-alkyl, $R_3$ is a hydroxyl group, $R_5$ is $C_1$-$C_3$-alkyl and m is the number 1.

**4.** The process as claimed in claim 1, which comprises preparing cis-(±)-2-(2-chlorophenyl)-5,7-dihydroxy-8-[4'-(3'-hydroxy-1'-methyl)-piperidinyl]-4H-1-benzopyran-4-one and pharmacologically acceptable acid addition salts thereof.

**5.** The process as claimed in claim 1, which comprises preparing cis-(-)-2-(2-chlorophenyl)-5,7-dihydroxy-8-[4'-(3'-hydroxy-1'-methyl)-piperidinyl]-4H-1-benzopyran-4-one and pharmacologically acceptable acid addition salts thereof.

**6.** The process as claimed in claim 1, which comprises preparing cis-(-)-2-phenyl-5,7-dihydroxy-8-[4'-(3'-hydroxy-1'-methyl)-piperidinyl]-4H-1-benzopyran-4-one and pharmacologically acceptable acid addition salts thereof.

**7.** The process as claimed in claim 1, which comprises preparing cis-(±)-2-phenyl-5,7-dihydroxy-8-[4'-(3'-hydroxy-1'-methyl)-piperidinyl]-4H-1-benzopyran-4-one and pharmacologically acceptable acid addition salts thereof.

**8.** The process as claimed in claim 1, which comprises preparing cis-(±)-2-(p-fluorophenyl)-5,7-dihydroxy-8-[4'-(3'-hydroxy-1'-methyl)-piperidinyl]-4H-1-benzopyran-4-one and pharmacologically acceptable acid addition salts thereof.

**9.** The process as claimed in claim 1, which comprises preparing cis-(±)-2-(2-pyridyl)-8-[4'-(3'-hydroxy-1'-methyl)-piperidinyl]-4H-1-benzopyran-4-one and pharmacologically acceptable acid addition salts thereof.

**10.** The use of a compound of the formula I of claim 1 for the preparation of a medicament having an antiinflammatory and/or immunomodulating action.

**Revendications**

**Revendications pour les Etats contractants suivants : BE, CH, DE, FR, GB, IT, LI, LU, NL, SE**

1. Composés de formule I, sous forme cis

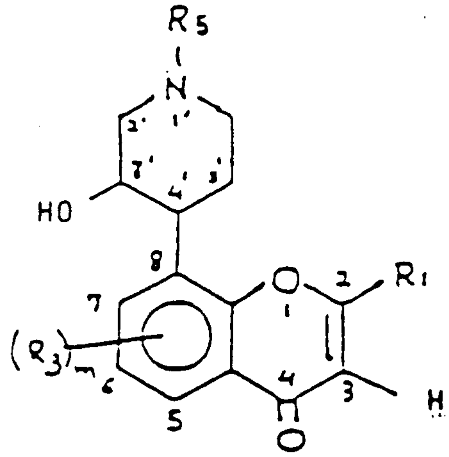

ainsi que leurs sels d'addition d'acide pharmacologiquement acceptables et leurs isomères optiques, formule dans laquelle:
$R_1$ représente un groupe alkyle en $C_{1-6}$, non substitué ou substitué par des atomes d'halogène ou des groupes hydroxyle ou carboxyle, un groupe pyridyle ou phényle, éventuellement substitués une ou plusieurs fois par des atomes d'halogène ou des groupes alcoxy en $C_{1-4}$, nitro, hydroxyle, carbonyle, amino, pyridyle ou trifluorométhyle,
$R_3$ représente un groupe hydroxyle ou alcoxy en $C_{1-4}$,
$R_5$ représente l'atome d'hydrogène ou un groupe alkyle en $C_{1-6}$ ou cycloalkyl ($C_{3-6}$)-alkyle($C_{1-4}$),
m est un nombre entier valant de 0 à 3,
à l'exception de toutes les formes du composé cis-5,7-dihydroxy-2-méthyl-8-[4'-(3'-hydroxy-1'-méthyl)-pipéridinyl]-4H-1-benzopyran-4-one.

2. Composés selon la revendication 1, dans lesquels $R_1$ et $R_5$ ont les significations indiquées, $R_3$ est le groupe hydroxyle et m vaut 2.

3. Composés selon la revendication 1, dans lesquels $R_1$ représente l'atome d'hydrogène ou un groupe alkyle en $C_{1-3}$, $R_3$est le groupe hydroxyle, $R_5$ est un groupe alkyle en $C_{1-3}$ et m vaut 2.

4. Le composé cis-(±)-2-(2-chlorophényl)-5,7-dihydroxy-8-[4'-(3'-hydroxy-1'-méthyl)pipéridinyl]-4H-1-benzopyran-4-one ainsi que ses sels d'addition d'acide pharmacologiquement acceptables.

5. Le composé cis-(-)-2-(2-chlorophényl)-5,7-dihydroxy-8-[4'-(3'-hydroxy-1'-méthyl)pipéridinyl]-4H-1-benzopyran-4-one ainsi que ses sels d'addition d'acide pharmacologiquement acceptables.

6. Le composé cis-(-)-2-phényl-5,7-dihydroxy-8-[4'-(3'-hydroxy-1'-méthyl)pipéridinyl]-4H-1-benzopyran-4-one ainsi que ses sels d'addition d'acide pharmacologiquement acceptables.

7. Le composé cis-(±)-2-phényl-5,7-dihydroxy-8-[4'-(3'-hydroxy-1'-méthyl)pipéridinyl]-4H-1-benzopyran-4-one ainsi que ses sels d'addition d'acide pharmacologiquement acceptables.

8. Le composé cis-(±)-2-(p-flourophényl)-5,7-dihydroxy-8-[4'-(3'-hydroxy-1'-méthyl)pipéridinyl]-4H-1-benzopyran-4-one ainsi que ses sels d'addition d'acide pharmacologiquement acceptables.

9. Le composé cis-(±)-2-(2-pyridyl)-8-[4'-(3'-hydroxyl-1'-méthyl)pipéridinyl]-4H-1-benzopyran-4-one ainsi que ses sels d'addition d'acide pharmacologiquement acceptables.

10. Procédé de préparation de composés de formule I selon la revendication 1

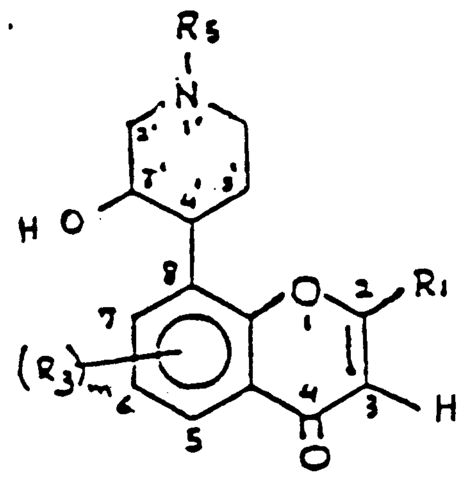

I

dans laquelle $R_1$ à $R_5$ et m ont les significations indiquées dans la revendication 1, ainsi que de leurs sels d'addition d'acide pharmacologiquement acceptables et de leurs isomères optiques, caractérisé en ce qu'on fait réagir un composé de formule XII

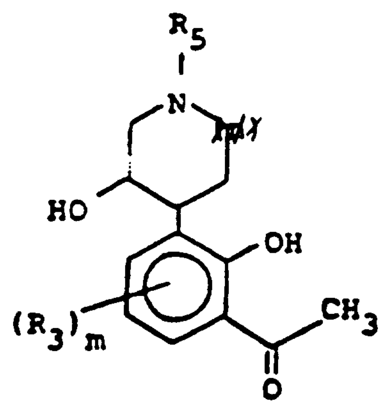

XII

dans laquelle $R_3$, $R_5$ et m ont les significations indiquées, avec un métal alcalin ou un hydrure de métal alcalin et un ester d'alyyle d'un acide, de formule $R_1$-COO-alkyl, où $R_1$ a la signification indiquée à propos de la formule I, pour obtenir une dicétone de formule XIII

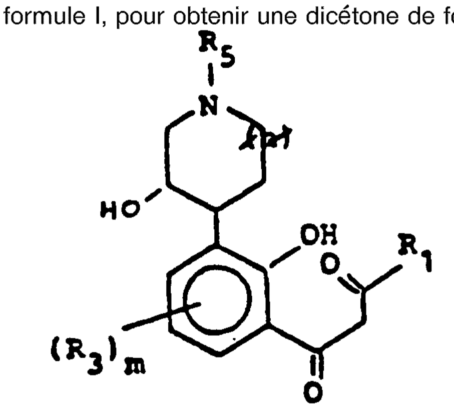

X III

on cyclise le composé obtenu en le faisant réagir avec un acide minéral, ce qui fournit un composé de formule I, dans laquelle $R_1$, $R_3$, $R_5$ et m ont les significations indiquées, on déméthoxyle éventuellement le composé de formule I en un composé de formule I dans laquelle $R_3$ est hydroxy et m a la signification indiquée, et on fait réagir éventuellement avec du bromure de cyanogène le composé de formule I, où $R_5$ est $CH_3$, après protection des groupes hydroxyle, puis on traite le composé obtenu par un réactif acide ou alcalin, pour obtenir un composé de formule I où $R_5$ est l'atome d'hydrogène,

on fait réagir éventuellement un composé de formule I, dans laquelle $R_5$ est l'atome d'hydrogène, avec des réactifs électrophiles appropriés, tels que des halogénures, des chlorures d'acide, des tosylates ou des énones, pour obtenir des composés de formule I où $R_5$ représente un groupe alkyle en $C_{1-6}$ ou cycloalkyl($C_{3-6}$)-alkyle($C_{1-4}$) substitué ou non substitué.

**11.** Médicaments, caractérisés en ce qu'ils contiennent un composé de formule I selon la revendication 1 ou un sel d'addition d'acide pharmacologiquement acceptable de ce composé.

**12.** Utilisation d'un composé de formule I selon la revendication 1 pour la fabrication d'un médicament à activité antiinflammatoire et/ou immunorégulatrice.

**Revendications pour les Etats contractants suivants : AT, ES, GR**

**1.** Procédé de préparation de composés de formule I, sous forme cis

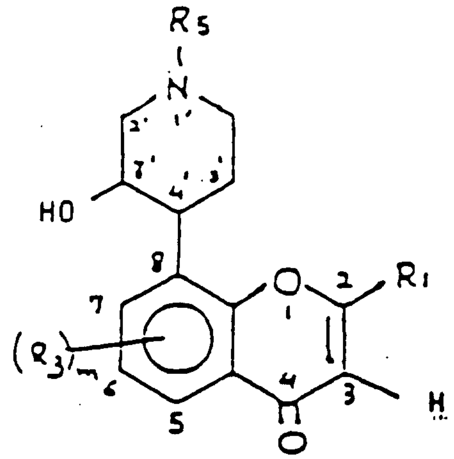

ainsi que de leurs sels d'addition d'acide pharmacologiquement acceptables et de leurs isomères optiques,
formule dans laquelle:
$R_1$ représente un groupe alkyle en $C_{1-6}$, non substitué ou substitué par des atomes d'halogène ou des groupes hydroxyle ou carboxyle, un groupe pyridyle ou phényle, éventuellement substitués une ou plusieurs fois par des atomes d'halogène ou des groupes alcoxy en $C_{1-4}$, nitro, hydroxyle, carbonyle, amino, pyridyle ou trifluorométhyle,
$R_3$ représente un groupe hydroxyle ou alcoxy en $C_{1-4}$,
$R_5$ représente l'atome d'hydrogène ou un groupe alkyle en $C_{1-6}$ ou cycloalkyl($C_{3-6}$)-alkyle($C_{1-4}$),
m est un nombre entier valant de 0 à 3,
à l'exception de toutes les formes du composé cis-5,7-dihydroxy-2-méthyl-8-[4'-(3'-hydroxy-1'-méthyl)-pipéridinyl]-4H-1-benzopyran-4-one,
caractérisé en ce qu'on fait réagir un composé de formule XII

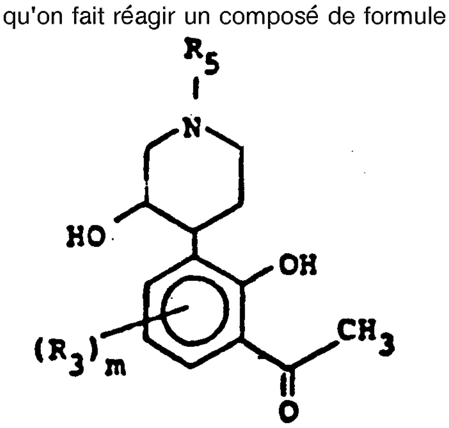

XII

dans laquelle $R_3$, $R_5$ et m ont les significations indiquées, avec un métal alcalin et un ester d'alkyle d'un acide, de formule $R_1$-COO-alkyl, où $R_1$ a la signification indiquée à propos de la formule I, pour obtenir une dicétone de formule XIII

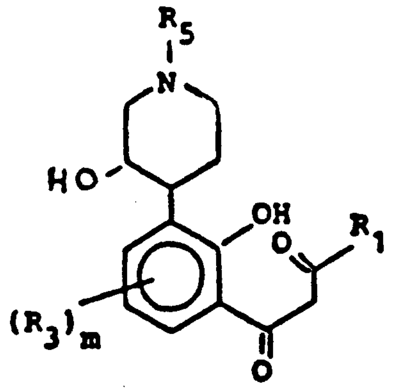

XIII

on cyclise le composé obtenu en le faisant réagir avec un acide minéral, ce qui fournit un composé de formule I, dans laquelle $R_1$, $R_3$, $R_5$ et m ont les significations indiquées, on déméthoxyle éventuellement le composé de formule I en un composé de formule I dans laquelle $R_3$ est hydroxy et m a la signification indiquée, et on fait réagir éventuellement avec du bromure de cyanogène le composé de formule I, où $R_5$ est $CH_3$, après protection des groupes hydroxyle, puis on traite le composé obtenu par un réactif acide ou alcalin, pour obtenir un composé de formule I où $R_5$ est l'atome d'hydrogène,
on fait réagir éventuellement un composé de formule I, dans laquelle $R_5$ est l'atome d'hydrogène, avec des réactifs électrophiles appropriés, tels que des halogénures, des chlorures d'acide, des tosylates ou des énones, pour obtenir des composés de formule I où $R_5$ représente un groupe alkyle en $C_{1-6}$ ou cycloalkyl($C_{3-6}$)-alkyle($C_{1-4}$) substitué ou non substitué.

2. Procédé selon la revendication 1, caractérisé en ce que $R_1$ et $R_5$ ont les significations indiquées, $R_3$ est le groupe hydroxyle et m vaut 2.

3. Procédé selon la revendication 1, caractérisé en ce que $R_1$ représente l'atome d'hydrogène ou un groupe alkyle en $C_{1-3}$, $R_3$ est le groupe hydroxyle, $R_5$ est un groupe alkyle en $C_{1-3}$ et m vaut 1.

4. Procédé selon la revendication 1, caractérisé en ce qu'on prépare le composé cis-(±)-2-(2-chlorophényl)-5,7-dihydroxy-8-[4'-(3'-hydroxy-1'-méthyl)pipéridinyl]-4H-1-benzopyran-4-one ou ses sels d'addition d'acide pharmacologiquement acceptables.

5. Procédé selon la revendication 1, caractérisé en ce qu'on prépare le composé cis-(-)-2-(2-chlorophényl)-5,7-dihydroxy-8-[4'-(3'-hydroxy-1'-méthyl)pipéridinyl]-4H-1-benzopyran-4-one ou ses sels d'addition d'acide pharmacologiquement acceptables.

6. Procédé selon la revendication 1, caractérisé en ce qu'on prépare le composé cis-(-)-2-phényl-5,7-dihydroxy-8-[4'-(3'-hydroxy-1'-méthyl)pipéridinyl]-4H-1-benzopyran-4-one ou ses sels d'addition d'acide pharmacologiquement acceptables.

7. Procédé selon la revendication 1, caractérisé en ce qu'on prépare le composé cis-(±)-2-phényl-5,7-dihydroxy-8-[4'-(3'-hydroxy-1'-méthyl)pipéridinyl]-4H-1-benzopyran-4-one ou ses sels d'addition d'acide pharmacologiquement acceptables.

8. Procédé selon la revendication 1, caractérisé en ce qu'on prépare le composé cis-(±)-2-(p-fluorophényl)-5,7-dihydroxy-8-[4'-(3'-hydroxy-1'-méthyl)pipéridinyl]-4H-1-benzopyran-4-one ou ses sels d'addition d'acide pharmacologiquement acceptables.

9. Procédé selon la revendication 1, caractérisé en ce qu'on prépare le composé cis-(±)-2-(2-pyridyl)-8-[4'-(3'-hydroxy-1'-méthyl)pipéridinyl]-4H-1-benzopyran-4-one ou ses sels d'addition d'acide pharmacologiquement acceptables.

10. Utilisation d'un composé de formule I selon la revendication 1 pour la fabrication d'un médicament à activité antiinflammatoire et/ou immunorégulatrice.

FIG. 1

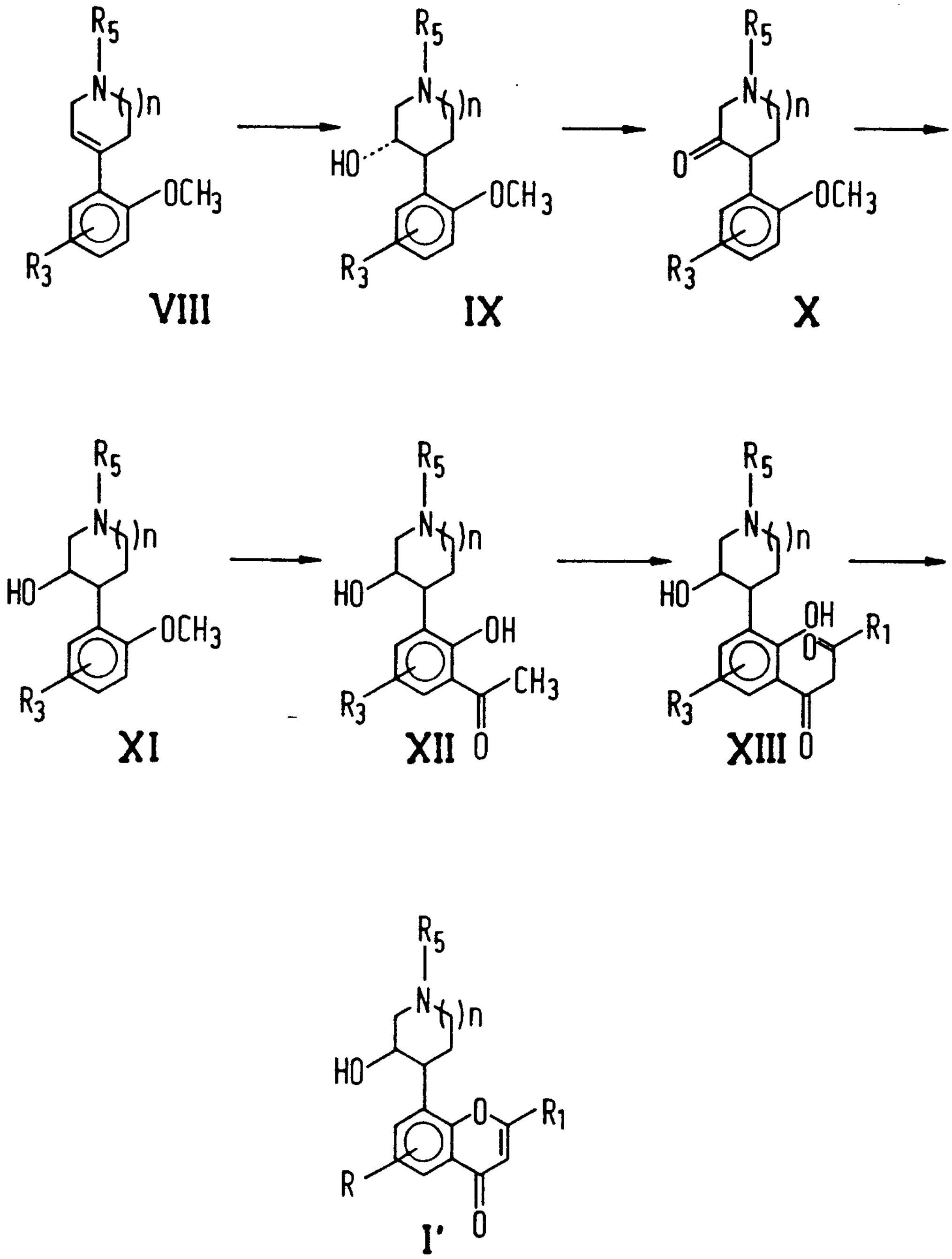

VIII IX X

XI XII XIII

I'

FIG. 2

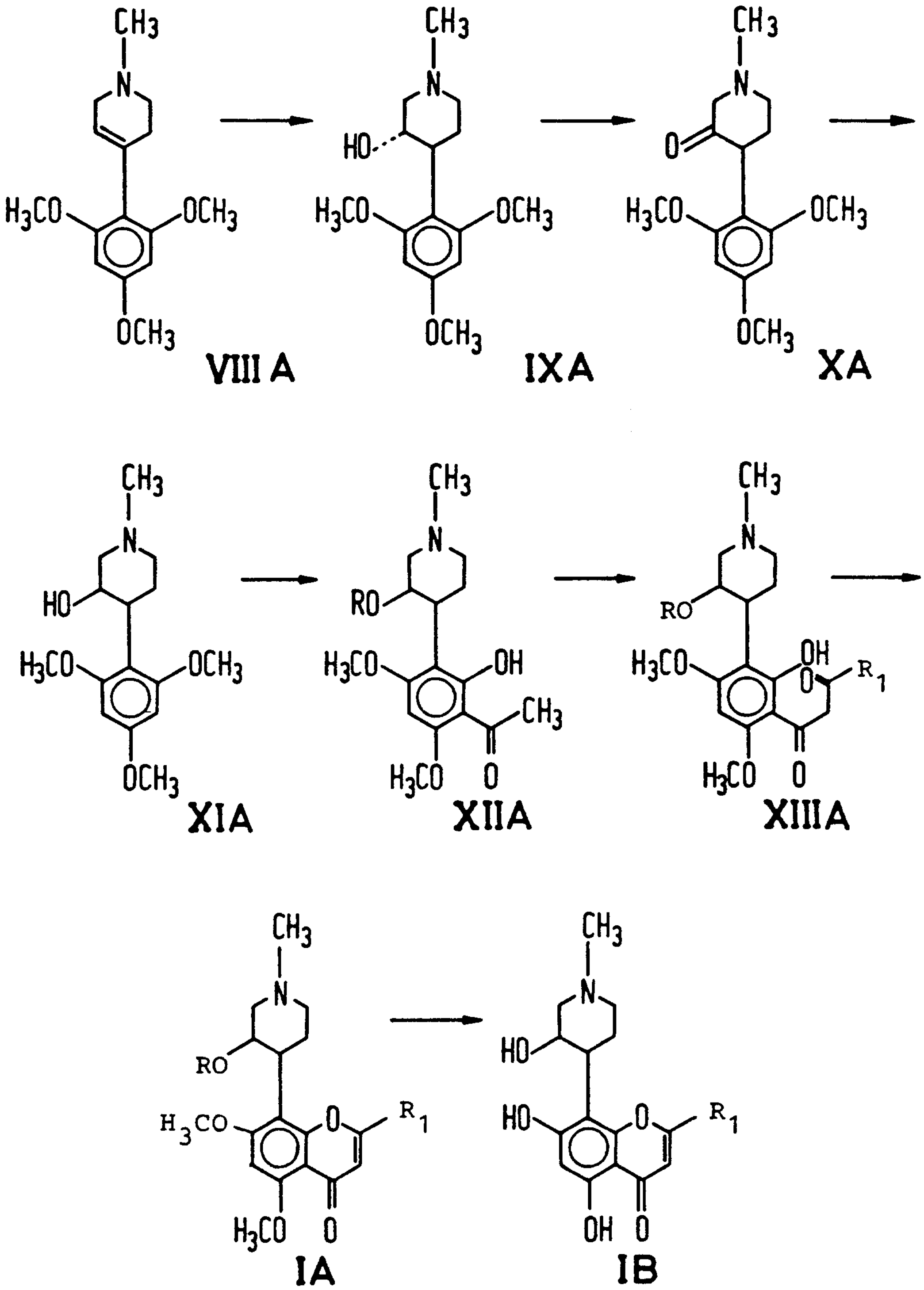

VIII A IXA XA

XIA XIIA XIIIA

IA IB

FIG.3

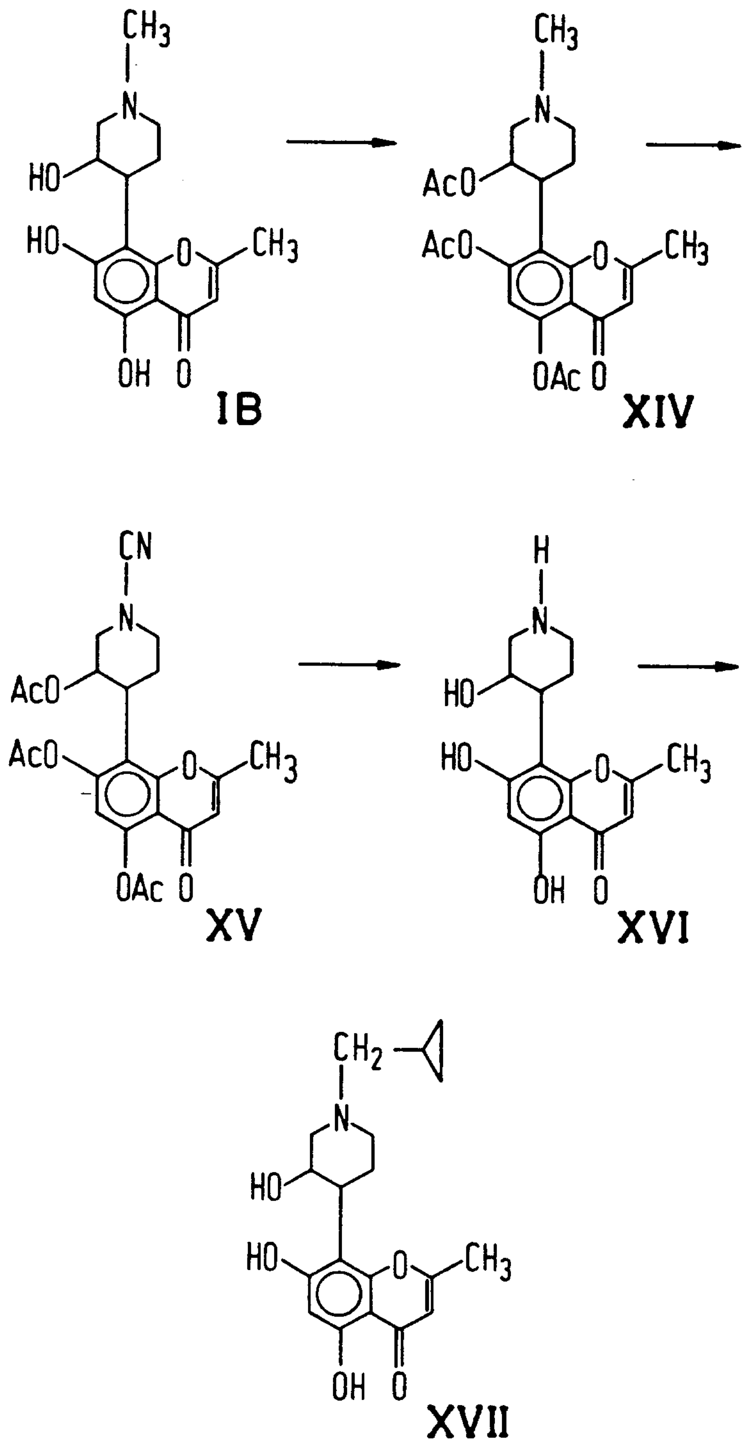

IB

XIV

XV

XVI

XVII